# EUROPEAN PATENT APPLICATION

(11) **EP 3 903 593 A1**
(43) Date of publication of application: **03.11.2021**
(21) Application number: 20382361.2
(22) Date of filing: 30.04.2020
(51) Int. Cl.: A23D 7/005, A23D 7/00, A23L 29/10, A23L 29/269

(54) **STRUCTURED FAT SYSTEM WITH REDUCED CONTENT IN OR FREE FROM TRANS AND SATURATED FATTY ACIDS AND USES THEREOF FOR THE PREPARATION OF FOODSTUFFS**

(71) Applicant: Cubiq Foods, S.L., 08401 Granollers (ES)
(72) Inventor: MONTEFELTRO, Andrés Pablo, E-08401 Granollers (ES); LARA CAMBIL, Armando, E-08401 Granollers (ES); REVILLA SÁNCHEZ, Raquel, E-08401 Granollers (ES)
(74) Representative: ABG Intellectual Property Law, S.L.

(57) **Abstract**

The invention relates to a structured fat system for use in food applications. The invention also relates to the use of a structured fat system for reducing the trans- and saturated fat content of a food product.

## Description

### FIELD OF THE INVENTION

The invention relates to a structured fat system for use in food applications. The invention also relates to the use of a structured fat system for reducing or eliminating the trans- and saturated fat content of a food product.

### BACKGROUND OF THE INVENTION

Solid fat systems are useful in many food applications in order to provide structure and stability. Solid fat systems contain lipid in solid form, in order to have the required functionality.

Nowadays, many oils are made solid through hydrogenation. Hydrogenation is a process commonly used to treat vegetable oils in order to increase their functionality by making them harder and of a comparable texture to butter for example. This process increases the saturated fatty acid content of the oil. Saturated fatty acids are fatty acids which do not contain any double bonds between the carbon atoms of the fatty acid chain. During the hydrogenation process, trans-fatty acids are also formed. Trans-fatty acids are unsaturated fatty acids in which the hydrogen atoms of a double-bond, are located on opposite sides of the molecule. Generally they are only found in low amounts in naturally occurring oils and fats. A trans-fat is an unsaturated fat with trans-isomer fatty acids.

Traditionally, the food industry is using solid fat systems based on animal fats (like butter or lard) which are rich in saturated fatty acids, harder vegetable oils (like palm oil) which are rich in saturated fatty acids and hydrogenated vegetable oils (from soybean or sunflower oil for example) which can be rich in trans fatty acids and saturated fatty acids. These hydrogenated oils have been subject to intensive research and studies have shown that the excessive consumption of these types of fats is one of the main causes of modern diseases such as cardiovascular diseases, obesity and some types of cancer. Industry is put more and more under pressure to reduce the amount of unhealthy fats in all kind of food applications. With this problem in mind, a lot of systems have been developed to replace trans-fat and/or saturated fat in food applications; some of them being entirely free of fat. Most of them have a paste-like structure that mimics the structure of fat. In general, these fat replacers are indicated in the replacement of fat for a very limited range of food applications.

Fat replacement is a difficult issue because fat plays a very important role in the manufacture and in the organoleptic properties of food. It also imparts the final aspect of many food applications. In fillings, coatings and spreads, fat plays the role of plasticizer and tenderizer and confers the right texture to the filling, coating or spread and to the final food product as a whole. It is desired that the fat does not leak out of the filling and does not separate from the filling, coating or spread.

One of the properties of oils is that they are free of trans-fatty acids and free of saturated fatty acids. Unfortunately, oils do not have the necessary structure for imparting a specific texture to food applications. Oils are difficult to use in fillings, coatings or spreads because the risk of leaking out.

There is thus a need for providing a saturated and trans-fat replacement system based on oil, that can be used in food and cosmetic applications, and which mimic the properties of the traditional fat or fat system.

### SUMMARY OF THE INVENTION

In one aspect, the invention relates to a method for obtaining a structured oil in the form of an oil-in-water (o/w) emulsion comprising the steps of
(i) Contacting an oil with an emulsifier and at least one hydrocolloid, said emulsifier and said at least one hydrocolloid being provided in aqueous solution and
(ii) Processing the mixture obtained in step (i) under conditions adequate for the dispersion of the oil in the aqueous solution to obtain the o/w emulsion.

In another aspect, the invention relates to a structured oil system in the form of an o/w emulsion comprising a lipid phase (o) and a water phase (w), the lipid phase being dispersed inside the water phase wherein the oil phase is stabilized in the water phase by an emulsifier and at least a first hydrocolloid dissolved or dispersed in the water phase.

In another aspect, the invention relates to foodstuff comprising a structured oil obtained by a method of the invention or comprising the structured oil according to the invention.

In another aspect, the invention relates to the use of the structured oil according to the invention for reducing the trans- or saturated fat content of a foodstuff.

In another aspect, the invention relates to a process for the manufacture of a foodstuff characterized in that it comprises a step of mixing a structured oil according to the invention with one or more edible ingredients.

In another aspect, the invention relates to a cosmetic composition comprising a structured oil according to the invention and one more cosmetically-acceptable carriers.

### LEGEND TO FIGURE

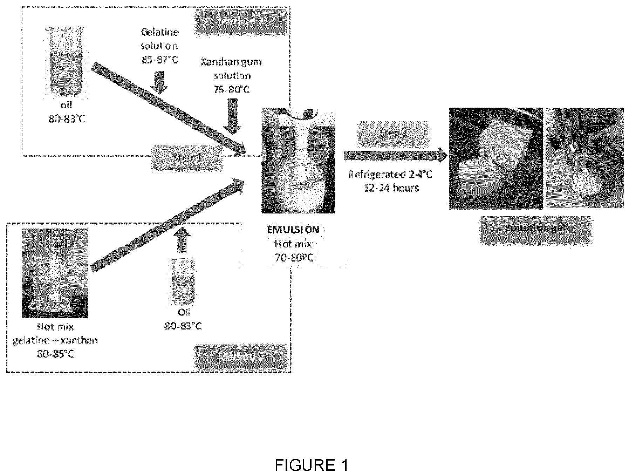

Figure 1 shows a scheme of the method for preparing the structured oil according to the invention.

### DETAILED DESCRIPTION OF THE INVENTION

### Method for the preparation of structured fat

The authors of the present invention have developed a method which allows the preparation of a new healthy structured fat which can be used to substitute fat with high content in saturated fatty acids, for example palm oil, coconut oil, hydrogenated oils, lard, tallow, etc. This structured fat shows similar properties to usual fat used in food industry but with a healthier profile of the lipidic components because it has a high content in unsaturated fatty acids or for their beneficial properties for our health.

In a first aspect, the invention relates to a method for obtaining a structured oil in the form of an oil-in-water (o/w) emulsion comprising the steps of
(i) Contacting an oil with an emulsifier and at least one hydrocolloid, said emulsifier and said at least one hydrocolloid being provided in aqueous solution and
(ii) Processing the mixture obtained in step (i) under conditions adequate for the dispersion of the oil in the aqueous solution to obtain the o/w emulsion.

The term "emulsion", as used herein, refers to a mixture of two or more liquids that are immiscible, in which one liquid (the dispersed phase) is dispersed in the other (the continuous phase). The emulsion which forms the structured oil according to the present invention is an oil-in-water (o/w) emulsion. As used herein, the term "oil-in-water (o/w) emulsion" refers to an emulsion in which the dispersed phase is an oil and in which the continuous phase is an aqueous solution.

The words "oil" or "fat" are used interchangeably herein, and refer to edible triacylglycerides of fatty acids. The person of average skill in the art will know that for such emulsion products to be spreadable (e.g. as a "spread") and in order to satisfy criteria such as health, mouthfeel and appearance, at least part of the continuous (lipid) phase (the fat and oil together) needs to be solid at room temperature. Still, for health reasons part of the continuous phase is preferably oil.

Naturally occurring vegetable oils, fish oils, extraction oils or fats or oils from animal or plant planktons, or fats or oils obtained synthetically contain a polyunsaturated fatty acid in the form of triglyceride, which can be directly used in the present invention. These may be appropriately selected depending upon the purposes. Since those having a higher content of the polyunsaturated fatty acids are more desired if some physiological effects are expected, the synthetic fat or oil, and fish oils and extraction oils or fats or oils from plant planktons can be preferably utilized.

Oils that can be used in the process for structuration according to the present invention can be classified according to their composition or according to the origin.

When classified according to their composition, the oils are defined by defining the alcohol and the fatty acid constituents that result in the oil upon esterification.

As the constituent alcohol, the term includes glycerol as well as any fatty alcohol and amino alcohol provided that is adequate for human or animal consumption. Suitable fatty alcohols that can be used in the oils according to the present invention include, without limitation, tert-Butyl alcohol, tert-Amyl alcohol, 3-Methyl-3-pentanol, 1-Heptanol (enanthic alcohol), 1-Octanol (capryl alcohol), Pelargonic alcohol (1-nonanol), 1-Decanol (decyl alcohol, capric alcohol), Undecyl alcohol (1-undecanol, undecanol, Hendecanol), Lauryl alcohol (dodecanol, 1-dodecanol), Tridecyl alcohol (1-tridecanol, tridecanol, isotridecanol), Myristyl alcohol (1-tetradecanol), Pentadecyl alcohol (1-pentadecanol, pentadecanol), Cetyl alcohol (1-hexadecanol), Palmitoleyl alcohol (cis-9-hexadecen-1-ol), Heptadecyl alcohol (1-n-heptadecanol, heptadecanol), Stearyl alcohol (1-octadecanol), Oleyl alcohol (1-octadecenol), Nonadecyl alcohol (1-nonadecanol), Arachidyl alcohol (1-eicosanol), Heneicosyl alcohol (1-heneicosanol), Behenyl alcohol (1-docosanol), Erucyl alcohol (cis-13-docosen-1-ol), Lignoceryl alcohol (1-tetracosanol), Ceryl alcohol (1-hexacosanol), 1-Heptacosanol, Montanyl alcohol, cluytyl alcohol, or 1-octacosanol, 1-Nonacosanol, Myricyl alcohol, melissyl alcohol, or 1-triacontanol, 1-Dotriacontanol (Lacceryl alcohol), Geddyl alcohol (1-tetratriacontanol). Suitable amino alcohols include sphingosine.

The term "fatty acid" as used herein refers to an organic compound having a (C4-C28) hydrocarbon chain possessing a terminal carboxylic acid group (i.e., -COOH), which is either saturated or unsaturated. Saturated fatty acids are composed of only C-C single bonds, whereas unsaturated fatty acids have one or more C-C double bonds. The expression "trans fatty acid" refers to unsaturated fatty acids in which the two hydrogen atoms of a double bond are in trans configuration, i.e. one on each side of the double bond.

As the constituent fatty acid, a saturated or unsaturated fatty acid having the number of carbon atoms of preferably 10 or more, more preferably 12 or more, and even more preferably 14 or more is preferred, from the viewpoint of interfacial modification of the polyunsaturated fatty acid and the basic amino acid and/or the basic peptide. Specific examples of the saturated fatty acids forming part of the oils which form the structured oil of the invention include capric acid, lauric acid, myristic acid, pentadecylic acid, palmitic acid, margaric acid, stearic acid, nonadecanoic acid, arachidic acid, behenic acid, and isomers thereof, and the like. Preferred fatty acids are unsaturated fatty acids such as palmitoleic acid, oleic acid, vaccenic acid, linoleic acid, eicosadienoic acid, docosadienoic acid, linolenic acid, ricinoleic acid, arachidonic acid, erucic acid, and isomers thereof, condensates thereof, and isomers of the condensates, and the like. Among them, the unsaturated or saturated fatty acid preferably includes myristic acid, palmitic acid, stearic acid, oleic acid, ricinoleic acid, linoleic oil, arachidonic acid enriched oil, docosahexaenoic acid (DHA) enriched oil, eicosapentaenoic acid (EPA) and isomers thereof, condensates thereof, and isomers of the condensates.

In preferred embodiments, the fatty acid is an omega-3 fatty acid or an omega-6 fatty acid.

As used herein, "omega-3 fatty acid" refers to any polyunsaturated fatty acid having a first double bond as the third carbon-carbon bond from the terminal methyl end of its carbon chain. In particular embodiments, the omega-3 fatty acid may comprise a long chain omega-3 fatty acid. As used herein, "omega-6 fatty acid" refers to any polyunsaturated fatty acid having a first double bond as the sixth carbon-carbon bond from the terminal methyl end of its carbon chain.

Suitable omega-3 fatty acids for use in embodiments of the present application can be derived from algae, fish, animals, plants, or combinations thereof, for example. Examples of suitable omega-3 fatty acids include, but are not limited to, linolenic acid, alpha-linolenic acid, eicosapentaenoic acid, docosahexaenoic acid, stearidonic acid, eicosatetraenoic acid and combinations thereof. In some embodiments, suitable omega-3 fatty acids can be provided in fish oils, (e.g., menhaden oil, tuna oil, salmon oil, bonito oil, and cod oil), microalgae omega-3 oils or combinations thereof. In particular embodiments, suitable omega-3 fatty acids may be derived from commercially available omega-3 fatty acid oils, such as Microalgae DHA oil (from Martek, Columbia, MD), OmegaPure (from Omega Protein, Houston, TX), Marinol C-38 (from Lipid Nutrition, Channahon, IL), Bonito oil and MEG-3 (from Ocean Nutrition, Dartmouth, NS), Evogel (from Symrise, Holzminden, Germany), Marine Oil, from tuna or salmon (from Arista Wilton, CT), OmegaSource 2000, Marine Oil, from menhaden and Marine Oil, from cod (from OmegaSource, RTP, NC).

Suitable omega-6 fatty acids include, but are not limited to, linoleic acid, gamma-linolenic acid, dihommo-gamma-linolenic acid, arachidonic acid, eicosadienoic acid, docosadienoic acid, adrenic acid, docosapentaenoic acid and combinations thereof.

In one embodiment, the oil is substantially free of trans fatty acids. The edible emulsion or structured fat of the invention is "substantially free of trans fatty acids". This means that the emulsion contains less than 0.1 wt percent of fatty acids; with respect to the total weight of the emulsion. The content of trans fatty acids can be easily determined by the skilled in the art using well-known techniques, such as gas chromatography.

Suitable oils for use according to the present invention include, when defined according to their composition, conjugated linoleic oil, arachidonic acid enriched oil, docosahexaenoic acid (DHA) enriched oil, eicosapentaenoic acid (EPA) enriched oil, palm stearic acid, omega-3 rich oils, which include flaxseed oil and algal oil, such as oils from *Schizochytrium* sp.

Suitable oils for use according to the present invention include, when defined according to their origin, vegetable oil, algal oil, a microbial oil or fish oil. Suitable vegetable oils include, without limitation, palm oil, palm kernel oil, rapeseed oil, sunflower oil, coconut oil, canola oil, soybean oil, flaxseed oil, wheat germ oil, corn oil, rice oil, olive oil, cottonseed oil, safflower oil, sesame oil, argan oil, walnut oil, almond oil, babassu oil, shea butter, mango butter, cocoa butter, borage oil, black currant oil, sea-buckhorn oil, macadamia oil, saw palmetto oil, sea-buckhorn berry oil, macadamia oil, saw palmetto oil, rice bran oil and peanut oil. Suitable oils from algae or microalgae include oil obtained from *Schizochytrium* sp. Microbial oils include oils obtained from *Rhodococcus* sp. or *Yarrobia* sp. Suitable fish oils include cod liver oil, mackerel oil, salmon oil and the like.

The content of the polyunsaturated fatty acids in these fats and oils is preferably 1 percent by weight or more, more preferably 5 percent by weight or more, and even more preferably 10 percent by weight or more, and preferably 99 percent by weight or less, and more preferably 95 percent by weight or less. The content of the polyunsaturated fatty acids as used herein means a total content in a case of containing plural polyunsaturated fatty acids. Here, fat or oils having a content of a polyunsaturated fatty acid of from 20 to 90 percent by weight or so are commercially available, and these can be also used. In addition, a fat or oil or a synthetic oil derived from other raw materials may be added to a naturally occurring extraction fat or oil to adjust the content of the polyunsaturated fatty acid.

In addition, other fatty acids may be contained, within the range that still be acceptable for edible and/or cosmetic application such as caproic acid and caprylic acid, 12-hydroxystearic acid, 9-hydroxystearic acid, 10-hydroxystearic acid, hydrogenated castor oil fatty acid (fatty acids containing small amounts of stearic acid and palmitic acid besides 12-hydroxystearic acid), and the like.

The terms "hydrocolloid" and "hydrogel" are used interchangeably herein. A hydrocolloid refers to a colloid system in which water is the dispersion medium. A "colloid" herein refers to a substance that is microscopically dispersed throughout another substance. Therefore, a hydrocolloid herein can also refer to a dispersion, emulsion, mixture, or solution of the polysaccharide derivative in water or aqueous solution. Hydrocolloids are capable of increasing the viscosity of an aqueous medium to which they have been added.

Suitable hydrocolloids for use according to the present invention include gelatin, polymeric glycosaminoglycans, galactomanans, glucomanans, agar, carrageenan, alginate, natural gums, carboxymethyl cellulose, pectin, dextran, dextran derivatives, pullulan, xanthan, xyloglucan, starch, hyaluronic acid, guar gum, locust bean gum, gellan, carboxy-methyl-cellulose, acacia gum, polyethylene glycol, polypropylene glycol, poly(tetramethylene ether) glycol, and/or combinations thereof. In a preferred embodiment, the hydrocolloid is a polysaccharide. Suitable polysaccharides that can be used as hydrocolloids according to the present invention include, without limitation, alginates, agar-agar, carragenates, xantham gum, arabic gum, galactomanans, glucomanans, xanthan gum with galactomanans, gellan gum, konjac gum, carragenates, pectines, amidated pectines, native and modified starch as well as modified celluloses. The term carragenate includes any of the three commercial classes of carrageenan Kappa, Iota and Lambda. The term galactomannan includes, without limitation, polymers having mannose:galactose ratios of 1:1, 2:1, 3:1, 4:1, 5:1 such as fenugreek gum, (mannose:galactose ratio of ca. -1:1), guar gum (mannose:galactose ratio of ca. 2:1), tara gum (mannose:galactose ratio of ca. 3:1), locust bean gum or carob gum (mannose:galactose ratio of ca. 4:1) or cassia gum (mannose:galactose ratio of ca. 5:1). The term "galactomannan" includes straight-chain polymer, with a small amount of branching, which contain β-(1→4)-linked D-mannose and D-glucose in a ratio of 1.6:1 and a degree of branching of about 8% through β-(1→6)-glucosyl linkages. Suitable sources of galactomannan include, without limitation, konjac root or salep powder.

In a preferred embodiment, the first hydrocolloid is a polysaccharide. In yet another embodiment, the polysaccharide is xanthan gum.

The hydrocolloid content in the mixture is not particularly limiting as long as the aqueous solution comprising the hydrocolloid and the emulsifier form a o/w emulsion when combined with the oil in a method according to the present invention. In some embodiment, the concentration if hydrocolloid or of the mixture of hydrocolloids is lower than the concentration needed to acquire a solid or a semi-solid at the temperature at which the method of the invention is carried out. In some embodiment, the hydrocolloid content in the mixture is of between 0,1 and 20% (w/w). In additional embodiments, the hydrocolloid content in the mixture is of between 0,1 and 18% (w/w). In additional embodiments, the hydrocolloid content in the mixture is of between 0,1 and 16% (w/w). In additional embodiments, the hydrocolloid content in the mixture is of between 0,1 and 14% (w/w). In additional embodiments, the hydrocolloid content in the mixture is of between 0,1 and 12% (w/w). In additional embodiments, the hydrocolloid content in the mixture is of between 0,1 and 10% (w/w). In additional embodiments, the hydrocolloid content in the mixture is of between 0,1 and 8% (w/w). In additional embodiments, the hydrocolloid content in the mixture is of between 0,1 and 6% (w/w). In additional embodiments, the hydrocolloid content in the mixture is of between 0,1 and 4% (w/w). In additional embodiments, the hydrocolloid content in the mixture is of between 0,1 and 2% (w/w). In additional embodiments, the hydrocolloid content in the mixture is of between 0,1 and 1% (w/w). In additional embodiments, the hydrocolloid content in the mixture is of between 0,1 and 0,8% (w/w). In additional embodiments, the hydrocolloid content in the mixture is of between 0,1 and 0,6% (w/w). In additional embodiments, the hydrocolloid content in the mixture is of between 0,1 and 0,4% (w/w). In additional embodiments, the hydrocolloid content in the mixture is of between 0,1 and 0,3% (w/w). In additional embodiments, the hydrocolloid content in the mixture is of between 0,1 and 0,2% (w/w).

In some embodiment, the "hydrocolloid content" refers to the total content of hydrocolloids in the mixture. In other embodiments, the hydrocolloid content refers to the content of each hydrocolloid.

Emulsifier and emulgent are used interchangeably in the present invention to refer to a substance which stabilizes an emulsion by increasing its kinetic stability. In particular, once an emulsion has been established, the emulsifier minimizes phase separation so that the relevant discrete and continuous phases forming the emulsion are maintained.

Suitable emulsifiers for use in the present invention include, without limitation, proteins, monoglycerides and diglycerides, sucroesters and sucroglycerides, propylenglycol fatty acid esters, lactic acid esters of mono and diglycerides, polyglyceride fatty acid esters, organic acid mono- and diglycerides esters, fatty acid salts and lecithins.

Suitable proteins for use as emulsifiers in the methods according to the present invention include pea protein, albumin or soy protein. In a preferred embodiment, the emulsifier is a denatured polypeptide. In yet further embodiments, the denatured polypeptide is selected from gelatine, pea protein, denatured collagen, albumin, casein, serum protein, soybean protein and rice protein.

In yet another embodiment, the denatured polypeptide is selected from gelatine or pea protein.

The term "mono- and diglycerides" refers to a mixture of mono- and diglyceryl esters of long chain, saturated and unsaturated fatty acids that occur in food fats. They contain not less than 30% of a-monoglycerides and may also contain other isomeric monoglycerides, as well as di- and triglycerides, free glycerol, free fatty acids, soap and moisture. They are usually manufactured by the glycerolysis of edible fats and oils, but may also be prepared by esterification of fatty acids with glycerol, with or without molecular distillation of the product. Monoglycerides correspond to the food additive known as E471 according to the FAO/WHO Codex Food Categorization System. Diglycerides correspond to the ingredient known as E473 according to the FAO/WHO Codex Food Categorization System.

The mono- or diglycerol is not particularly limited and any of monoglycerols, diglycerols, polyglycerols, and the like can be used. In addition, the polyglycerol is preferably a polyglycerol of which hydroxyl value is 1,200 or less, and primary hydroxyl groups of all the hydroxyl groups is 50 percent by number or more. Among them, it is desired that the polyglycerol of which primary hydroxyl group is preferably 55 percent by number or more, and more preferably 60 percent by number or more, from the viewpoint of the effects on increasing compatibility between the polyunsaturated fatty acid-containing fat or oil and the basic amino acid and/or the basic peptide, and further the upper limit is, but not particularly limited thereto, 90 percent by number or less, preferably 80 percent by number or less, and more preferably 70 percent by number or less, in order to maximally exhibit the effects thereof. In addition, the hydroxyl value is preferably 1,100 or less, and more preferably 1,000 or less, from the viewpoint of being capable of adjusting hydrophilicity (HLB) of the polyglycerol fatty acid esters depending upon the applications. In addition, the hydroxyl value is preferably 770 or more, from the viewpoint of workability and easiness in esterification with fatty acids.

Monoglycerides suitable for use as emulsifiers in the methods according to the present invention include mono-staryl glycerol, mono-lauryl glycerol, mono-oleyl glycerol, etc. Diglycerides suitable for use according to the present invention include stearyl-oleyl-gliceryde, palmityl-oleiyl-glyceride, dioleyl-glyceride, disteary glycerol, etc.

Sucrose esters or sugar esters (correspond to the food additive known as E473 according to the FAO/WHO Codex Food Categorization System) are defined as mono-, di- and tri-esters of sucrose with fatty acids. Sucrose esters are prepared by reacting sucrose and methyl and ethyl esters of fatty acids or by extraction from sucroglycerides. Since sucrose has a total of eight hydroxyl groups (three are primary groups and five are secondary groups), compounds ranging from sucrose mono- to octa- fatty acids esters can be produced. Although fatty acids in the C8 to C22 range are suitable to esterify sucrose, long chain fatty acids of C14 to C18 are preferred. Such long chain fatty acids include sucrose laurate, sucrose palmitate, sucrose stearate and sucrose oleate. Sucrose esters that have one, two or three fatty acids, or mono, di, and triesters, are referred to as sucrose fatty acid esters (SFAEs). Other sucrose esters that have four or more, up to eight fatty acids esters, are referred to as sucrose polyesters (SPEs). Sucrose fatty acid esters with toxicological clearance have been approved for specific uses in the United States since 1983. The materials approved for food use are mixtures of mono-, di-, and triesters of palmitic and stearic acids. FDA and EC (European Community) standards require a minimum of 80% total content of mono-, di-, and triester to be food approved. Sucrose polyesters with four or more fatty acids are not approved for food use in the United States or EC.

Sucroglycerides (correspond to the food additive E474 according to the FAO/WHO Codex Food Categorization System) y consist of a mixture of mono- and di-esters of sucrose and fatty acids together with mono-, di- and triglycerides from the fat or oil.

Propylenglycol fatty acid esters (food additive E477) are defined propylene glycol esters of fatty acids are mixtures of propylene glycol mono- and diesters of saturated and unsaturated fatty acids derived from edible oils and fats (FAO-JEFCA definition). The products are produced either by direct esterification of propylene glycol with fatty acids or by transesterification of propylene glycol with oils or fats. When prepared by transesterification, the product may contain residual mono- and diglycerides and glycerol. The process may be followed by molecular distillation to separate the monoesters.

Lactic acid esters of mono- and diglycerides (food additive E472b) are defined as a mixture of glycerol esters of lactic acid and fatty acid of food fats (FAO-JEFCA definition). These compounds are characterized by the following structural formula: 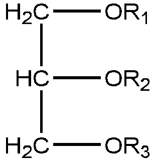 wherein R₁, R₂ and R₃ each may be a fatty acid moiety, a lactic acid moiety, or hydrogen (approximate composition) but wherein at least one of R₁, R₂ and R₃ is lactic acid The article of commerce may be further specified as to monoglyceride content, total lactic acid, acid value, saponification value, free fatty acid content, solidification point of the free fatty acids, iodine value, free glycerol content and water content.

Polyglycerol esters of fatty acids (food additive E475) are defined as mixed partial esters formed by reacting polymerized glycerols with edible fats, oils, or fatty acids (FAO-JEFCA definition). Minor amounts of mono-, di-, and triglycerides, free glycerol and polyglycerols, free fatty acids, and sodium salts of fatty acids may be present. The polyglycerols vary in degree of polymerization, which is specified by a number (such as tri-) that is related to the average number of glycerol residues per polyglycerol molecule. A specified polyglycerol consists of a distribution of molecular species characteristic of its nominal degree of polymerization. By varying the proportions as well as the nature of the fats or fatty acids to be reacted with the polyglycerols, a large and diverse class of products may be obtained. The article of commerce may be further specified as to saponification value, solidification point of the free fatty acids, iodine value, hydroxyl value and ash content.

Further emulsifiers include compounds belonging to the family of the organic acids mono- and diglycerides esters, which include citric and fatty acids esters of glycerol, acetic and fatty acids esters of glycerol and tartaric, acetic and fatty acid esters of glycerol.

Citric and fatty acids esters of glycerol (food additive E472c) are esters obtained by esterification of glycerol with citric acid and edible fatty acids, or by reaction of a mixture of mono- and diglycerides of edible fatty acid, with citric acid; consists of mixed esters of citric acid and edible fatty acids with glycerol; may contain minor parts of free fatty acids, free glycerol, free citric acid and mono- and diglycerides; may be wholly or partially neutralized with sodium hydroxide or potassium hydroxide (as declared on the label). Their structural formula is 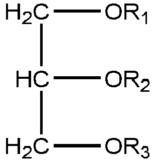 where at least one of R₁, R₂ or R₃ represents a citric acid moiety, one represents a fatty acid moiety and the remainder may represent citric acid, fatty acid or hydrogen.

Acetic and fatty acids esters of glycerol (food additive E472a) are, according to the FAO-JEFCA definition, mixed glycerol esters of acetic acid and fatty acids of food fats. Contains mono- and diesters of fatty acids with glycerol which is itself partially acetylated; may also contain free glycerol and free fatty acids. Their structural formula is 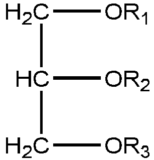 wherein R₁, R₂ and R₃ each may be a fatty acid moiety, - COCH3 or H but wherein at least one of R₁, R₂ and R₃ is -COCH₃.

Tartaric, acetic and fatty acid esters of glycerol, (food additive, E472e & f) are, according to the FAO/WHO definition, esters of glycerol with fatty acids of food fats, acetic acid and tartaric acid. It may contain small amounts of free glycerol, free fatty acids, free acetic acid, free tartaric acid and free glycerides. Their structural formula is: 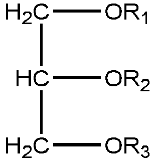 wherein one or two of the R groups is a fatty acid moiety and the other R groups are either a tartaric acid moiety, an acetic acid moiety, hydrogen- diacetylated tartaric acid moiety or monoacetylated tartaric acid moiety.

Calcium, potassium and sodium salts of fatty acid (food additive E470), FAO-JEFCA definition: These products consist of calcium, potassium or sodium salts of commercial myristic, oleic, palmitic, stearic, acids or mixtures of these acids from edible fats and oils.

The term "lecithin", as used herein, refers to any mixture of one or more glycerophospholipids. Lecithin can be obtained as a mixture of phosphatides and triglycerides by water-washing crude vegetable oils, and separating and drying the resulting hydrated gums. A refined product can be obtained by fractionating the mixture for acetone insoluble phospholipids and glycolipids remaining after removal of the triglycerides and vegetable oil by acetone washing. Alternatively, lecithin can be obtained from various commercial sources.

The term "glycerophospholipid" refers to a lipid containing glycerol bound by means of ester bonds to two fatty acid groups and to a phosphate group, said phosphate group optionally being bound through a phosphoester bond to another group such as a choline, ethanolamine, inositol or serine, or to lipids having a phosphocholine or phosphoethanolamine fragment bound through an ester bond to the 1-hydroxyl group of a ceramide (the ceramide being a sphingosine bound to a fatty acid). Examples of glycerophospholipids are phosphatidylcholine (PC), lysophosphatidylcholine (LPC), phosphatidylethanolamine (PE), phosphatidylinositol (PI), phosphatidylserine (PS), and phosphatidic acids. Preferably, the lecithin comprises one or more phospholipids selected from the group consisting of phosphatidylcholine, lysophosphatidylcholine, phosphatidylethanolamine, phosphatidylinositol, and phosphatidic acid. More preferably the lecithin comprises phosphatidylcholine, lysophosphatidylcholine, phosphatidylethanolamine, phosphatidylinositol, and phosphatidic acid.

In one embodiment, the method comprises the use of a single emulsifier and a single hydrocolloid. In other embodiment, the method comprises the use of an emulsifier and a first and second hydrocolloid. In yet another embodiment, the method comprises the use of an emulsifier, a first hydrocolloid, a second hydrocolloid and an additional hydrocolloid. Suitable emulsifiers, first hydrocolloids, second hydrocolloids and additional hydrocolloids are as defined above.

The emulsifier content in the mixture is not particularly limiting as long as the aqueous solution comprising the hydrocolloid and the emulsifier form a o/w emulsion. In some embodiment, the emulsifier content in the mixture is of between 0,1 and 10% (w/w). In additional embodiments, the emulsifier content in the mixture is of between 0,1 and 99,5% (w/w). In additional embodiments, the emulsifier content in the mixture is of between 0,1 and 9% (w/w). In additional embodiments, the emulsifier content in the mixture is of between 0,1 and 8% (w/w). In additional embodiments, the emulsifier content in the mixture is of between 0,1 and 6% (w/w). In additional embodiments, the emulsifier content in the mixture is of between 0,1 and 5% (w/w). In additional embodiments, the emulsifier content in the mixture is of between 0,1 and 4% (w/w). In additional embodiments, the emulsifier content in the mixture is of between 0,1 and 3% (w/w). In additional embodiments, the emulsifier content in the mixture is of between 0,1 and 2% (w/w). In additional embodiments, the emulsifier content in the mixture is of between 0,1 and 1% (w/w). In additional embodiments, the emulsifier content in the mixture is of between 0,1 and 0,5% (w/w). In additional embodiments, the emulsifier content in the mixture is of between 0,1 and 0,4% (w/w). In additional embodiments, the emulsifier content in the mixture is of between 0,1 and 0,3% (w/w). In additional embodiments, the emulsifier content in the mixture is of between 0,1 and 0,2% (w/w),

In some embodiment, the "emulsifier content" refers to the total content of emulsifier in the mixture. In other embodiments, the emulsifier content refers to the content of each emulsifier.

Since emulsifiers can also act as hydrocolloids, the method according to the present invention can be carried out using a molecule having both emulsifier and hydrocolloid properties. In this case, a second hydrocolloid and a third hydrocolloid can also be included. Suitable hydrocolloids having emulsifier activity include, without limitation, elatin, collagen peptides, albumin, casein, caseinate, gum arabic or acacia gum, xanthan gum, guar cum, tara gum, agar-agar, carrageenates and alginates. The second and optional third hydrocolloids need not act as emulsifiers. In one embodiment, the second hydrocolloid is a polysaccharide, preferably xanthan gum, alginate, agar agar or carrageenan. In another embodiment, when an additional hydrocolloid is added, the hydrocolloid is selected from the group consisting of guar gum, locust bean gum, xanthan gum or konjac.

In another embodiment, the oil, the emulsifier, the first, the second and any additional hydrocolloids are natural ingredients.

In the present description, what is meant by "natural ingredients" is ingredients of natural origin. These include ingredients which come directly from the field etc. They may also include ingredients which are the result of a physical or microbiological/enzymatic process (e.g. extraction, fermentation etc.). However, they do not include ingredients which are the result of a chemical modification process. In another embodiment, the natural ingredients derive from a non-animal source, preferably a plant source, an algal source or a microbial source.

In further embodiments, the method for obtaining a structured oil is carried out using a combination of emulsifier, first hydrocolloid, second hydrocolloid and additional hydrocolloid as defined in Table 1

**Table 1: Suitable combinations of emulsifiers, first hydrocolloid, second hydrocolloid and additional hydrocolloid for use in the method according to the present invention.**

| Emulsifier | First hydrocolloid | Second hydrocolloid | Additional hydrocolloid |
|---|---|---|---|
| Gelatin | Xanthan gum | | |
| Gelatin | Xanthan gum | Guar gum | |
| Pea protein | Xanthan gum | Locust bean gum | |
| Pea protein | Xanthan gum | Guar gum | Locust bean gum |
| Pea protein | Alginate | Xanthan gum | |
| Pea Protein | Alginate | Xanthan gum | Guar gum |
| Pea protein | Agar agar | Xanthan gum | |
| Pea protein | Agar agar | Xanthan gum | Locust bean gum |
| Pea protein | Carragenan | Locust bean gum | |
| Pea protein | Carragenan | konjac | |
| Pea protein | Carragenan | Xanthan gum | Guar guam |
| Soybean protein | Carragenan | Locust bean gum | Xanthan gum |

Preferred combinations of oil phase, emulsifier and hydrocolloids according to the present invention are shown in Table 2:

**Table 2: Preferred compositions according to the invention. The emulsifier, the first hydrocolloid, the second hydrocolloid, the additional hydrocolloid if used and the flavouring agents are provided in aqueous solution. Preservatives and dyes will be added to aqueous or oil phase depending on their solubility in each phase.**

| Oil | Emulsifier | Hydrocolloid 1 | Hydrocolloid 2 | Additional hydrocolloid | Flavouring agentes | Antioxidants | Other (pH regulators and thickening agents), | Dyes |
|---|---|---|---|---|---|---|---|---|
| Sunflower oil | Gelatin | Xantham gum | | | Bovine flavour | Tocopherol | | |
| Sunflower oil | Gelatin | Xantham gum | | | | Tocopherol | | |
| Sunflower oil | Gelatin | Xantham gum | | | Chicken flavour | Tocopherol | | |
| Sunflower oil | Gelatin | Xantham gum | Guar gum | | | Tocopherol | | |
| Sunflower oil | Pea protein | Sodium alginate | Guar gum | Xantham gum | | | Calcium sulphate sodium citrate Citric acid | |
| Sunflower oil | Pea protein | Sodium alginate | Guar gum | Xantham gum | | | Calcium sulphate sodium citrate Citric acid | Carmi ne |
| Sunflower oil | Pea protein | Sodium alginate | Guar gum | Xantham gum | | Tocopherol | Calcium sulphate sodium citrate Citric acid | |
| Sunflower oil | Pea protein | Agar Agar | Guar gum | Xantham gum | | Tocopherol | | |
| Sunflower oil | Pea protein | Agar Agar | Locust bean gum | Xantham gum | | Tocopherol | | |
| Sunflower oil | Pea protein | Carragenan iota | Carragenan kappa | Locust bean gum | | Tocopherol | Potassium chloride, calcium lactate | |

In another embodiment, the method according to the present invention involves the use of one or more aqueous solutions or oil which comprises or comprise one or more food additives. It will be understood that. If the food additive is added as forming part of the oil, then it will be found predominantly in the oil phase of the resulting structured oil. If the food additive is added as forming part of one or more of the aqueous solutions, then it will be found predominantly in the aqueous phase of the resulting structured oil.

In a preferred embodiment, the food additive is selected from the group consisting of aroma or flavouring compounds, food dyes and sweeteners. Suitable food additives for use in the method of the invention are defined below in the context of the "structured oil" of the invention and include, without limitation, an antifreezing agent and/or a cryoprotective agents,

In another embodiment, the method according to the present invention involves the use of one or more aqueous solutions which, in addition to the emulsifier and hydrogel or hydrogels, further comprises or comprise an antifreezing agent and/or a cryoprotective agents, "aroma" or "flavouring compounds", term "food dye", and sweeteners.

In step (i), the method for obtaining a structured oil in the form of an oil-in-water (o/w) emulsion comprises contacting an oil with a emulsifier and at least one hydrocolloid, said emulsifier and said at least one hydrocolloid being provided in aqueous solution.

In one embodiment, the first step is carried out using a single hydrocolloid. In a more preferred embodiment, the emulsifier and the single hydrocolloid are provided in a first and second aqueous solutions which are consecutively added to the oil. The order of addition of components is not particularly limitative. In one embodiment, the first aqueous solution containing the emulsifier is added firstly to the oil and the second aqueous solution containing the hydrocolloid is added subsequently to the oil. In another embodiment, the second aqueous solution containing the hydrocolloid is added firstly to the oil and the first aqueous solution containing the emulsifier is added subsequently to the oil. In another embodiment, the oil is added to the aqueous solution containing the first hydrocolloid and the emulsifier. In another embodiment, the first and second aqueous solutions containing respectively, the first hydrocolloid and the emuslfier are combined and then the oil is added to the mixture resulting from the combination of hydrocolloid and emulsifier.

In a preferred embodiment, the water content in the mixture obtained in step (i) is between 30 and 99% (w/w). In another embodiment, the oil content in the mixture obtained in step (i) is between 0,1 and 70% (w/w). In yet another embodiment, the emulsifier content in the mixture obtained in step (i) is between 0,1 and 10% (w/w)., In another embodiment, the total hydrocolloid content in the mixture obtained in step (i) is between 0,1 and 20% (w/w).

In another preferred embodiment, the emulsifier and the single hydrocolloid are provided in a single aqueous solution which is added to the oil. In this case, in one embodiment, the emulsifier is a compound which is different to the first hydrocolloid. In another embodiment, the emulsifier and the second hydrocolloid are different compounds. In another the first and second hydrocolloids are different compounds. In yet another embodiment, the emulsified, the first and second hydrocolloids are different compounds.

When step (i) of the method according to the present invention is carried out in the presence of a first and a second hydrocolloids, the following options exist when incorporating the aqueous solutions containing the components to the oil:
- The emulsifier and the first and second hydrocolloids are provided in a first, second and third aqueous solutions which are consecutively added to the oil.
- the emulsifier is provided in a first aqueous solution and the first and second hydrocolloids are provided in a second aqueous solution which are added to the oil emulsifier
- The emulsifier, the first and the second hydrocolloids are provided in a single aqueous solution which is added to the oil
- The emulsifier and the first and second hydrocolloids are provided in a first, second and third aqueous solutions which are firstly mixed and to which the oil is subsequently added,
- the emulsifier is provided in a first aqueous solution and the first and second hydrocolloids are provided in a second aqueous solution which are firstly mixed and to which the oil is subsequently added.
- The emulsifier, the first and the second hydrocolloids are provided in a single aqueous solution to which the oil is added.

In one embodiment of the method according to the invention when two hydrocolloids are used, then the content of each hydrocolloid is of between 0,1 and 10% (w/w). In yet another embodiment, the ratio between the first and second hydrocolloid is of 0,1:4 to 4:0,1.

In a preferred embodiment, the water content in the mixture obtained in step (i) is between 30 and 99% (w/w). In another embodiment, the oil content in the mixture obtained in step (i) is between 0,1 and 70% (w/w). In yet another embodiment, the emulsifier content in the mixture obtained in step (i) is between 0,1 and 10% (w/w)., In another embodiment, the total hydrocolloid content in the mixture obtained in step (i) is between 0,1 and 20% (w/w) and wherein the content of each hydrocolloid is of between 0,1 and 10% (w/w).

In one embodiment, the oil in step (i) is provided at a temperature of about 80-83°C.

It will be understood that the conditions for forming the aqueous solutions containing the emulsifier, the first hydrocolloid or, as the case may be, the third hydrocolloid as well as the conditions used for the contacting of the aqueous solutions containing the emulsified and the hydrogel or hydrogel as the case may be are not particularly limitative of the scope of the invention as long as the emulsion is formed.

In a second step, the method for obtaining a structured oil in the form of an oil-in-water (o/w) emulsion comprises processing the mixture obtained in step (i) under conditions adequate for the dispersion of the oil in the aqueous solution to obtain the o/w emulsion.

Suitable methods for detecting the formation of the emulsion include, without limitation:
- Viscosity determination: the formation of the emulsion is accompanied by an increase in viscosity that the can be determined using widely available methods,
- Visual inspection: the formation of the emulsion can be determined by adding a hydrosoluble dye to the mixture so that, before the emulsion is formed, the due appears in the aqueous phase whereas once the emulsion is formed, it appears dispersed throughout the emulsion in the aqueous phase
- Microscopic examination: visualization of the oil droplet within the aqueous phase
- Conductivity determination: the formation of the emulsion is accompanied by an increase in conductivity,
- Detection of phase separation after centrifugation,
- Dilution: turbidity measurement after the addition of a small amount of the emulsion to a aqueous solvent.

All these methods are routine methods that can be found, for instance, in Hu et al. (Journal of Food and Drug Analysis, 2017, 25: 16-26), which is incorporated herein by reference in its entirety.

In some embodiments, the second step is carried out until the oil is stably dispersed within the aqueous phase.

"Stably dispersed", as used herein, means that the oil particles are dispersed as solids in the continuous fluid phase of the composition, i.e. the liquid hydrogel. The term "stably" preferably relates to the particles not sedimenting to the bottom of the composition or floating to the surface of the composition over typical time periods of storage.

In one embodiment, step (ii) is carried out by shearing at a speed ranging between 1000 to 10.000 rpm, between 2000 and 9000 rpm, between 3000 and 8000 rpm, between 4000 and 7000 rpm or between 5000 and 6000 rpm.

In another embodiment, step (ii) is carried out at a temperature of between 70-80°C.

In one embodiment, the method according to the invention further comprises a step wherein the structured oil obtained in step (ii) is processed under conditions adequate for the gelation of the structured oil.

The terms "solidification", "gelation" and "sol-gel transition" are used interchangeably herein to refer to the phase transition between the liquid state (or sol state) and the solid (or semi-solid) state, known as gel phase.

In one embodiment, the gelation is carried out by shifting the structured oil at a temperature which is below the gelation Temperature .

The term "gelation temperature," as used herein is defined as meaning the temperature below which a composition is in solid or semi-solid and above which the composition forms a liquid.

In another embodiment, gelation is carried out by "ionic gelation". The term "ionic gelation", as used herein, refers to complexation between oppositely charged molecules that, when combined, form a ionic complex which acquires an ordered structure. The "ionic gelation" requires the contacting of a negatively charged ion and a positively charged counterion. Preferably, the combination of aqueous solutions is done by dropwise addition of one aqueous solution to the other under constant stirring. In one preferred embodiment, the gelation step that can be applied to the structured oil obtained by the method of the invention involves the use of negatively charged macromolecules and soluble salts of polyvalent cations. In a more preferred embodiment, the gelation is carried out using alginate as negatively charged macromolecules and divalent calcium cations as counterions, which results in the formation of a ordered calcium alginate solution.

In still another embodiment, the gelation is carried out in response to changes in pH. Optionally, the gel-forming system undergoes gel-formation in response to a combined change in pH and temperature, such as a pH in the range of 6-8 and a temperature in the range of 35 to 40 degrees centigrade

Non-limiting examples of such gel-forming systems are illustrated in FIG. 4, and include those described in i) Macromol. Biosci., 2010, 10, 563-579, ii) J. Controlled Release, 2001, 73, 205-211, iii) Topics in tissue engineering-Smart Polymers, Vol. 3, 2007, Chapter 6, iv) Adv. Drug Delivery Rev., 2010, 62, 83-99, v) J. Controlled Release, 2003, 86, 253-265 and references cited therein.

The properties of pH responsive hydrogels are highly depending on the pKₐ of the ionizable moiety, the hydrophobic moieties in the polymer backbone, their amount and distribution. When ionizable groups become neutral-non-ionized-and electrostatic repulsion forces disappear within the polymer network, hydrophobic interactions dominate. The introduction of a more hydrophobic moiety can offer a more compact conformation in the uncharged state and a more accused phase transition. The hydrophobicity of these polymers can be controlled by the copolymerization of hydrophilic ionizable monomers with more hydrophobic monomers with or without pH-sensitive moieties, such as 2-hydroxyethyl methacrylate, methyl methacrylate and maleic anhydride.

In some embodiments, the gelation process is carried out until a gel is formed from the structured oil which has a viscosity of at least about 0.3 Pas, such as at least about 0.4 Pas (measured at 12 degrees centigrade, shear rate of 100 1/s). In another embodiment of the present invention gelation process is carried out until a gel is formed from the structured oil which has a viscosity of at least about 0.6, 0.8, 1.0, or 1.2 Pas (measured at 12 degrees centigrade, shear rate of 100 1/s). In one embodiment, the viscosity is about 0.3-2.0 Pas, such as about 0.4-1.8 Pas (measured at 12 degrees centigrade, shear rate of 100 1/s). In further embodiments, the gelation process is carried out until a gel is formed from the structured oil which has a viscosity of about 0.3-1.2, such as about 0.3-1.0 or about 0.4-0.6 Pas (measured at 12 degrees centigrade, shear rate of 100 1/s). In further embodiments, the gelation process is carried out until a gel is formed from the structured oil which has a viscosity of about 0.8-1.9 Pas, such as about 1.0-1.7 or about 1.2-1.5 Pas (measured at 12 degrees centigrade, shear rate of 100 1/s).

The gelation step is carried out until the gelled emulsion acquires sufficient strength Typically, strength is determined using the Bloom test. The test was originally developed and patented in 1925 by Oscar T. Bloom to measure the strength of a gel or gelatin. The test. The test determines the weight in grams needed by a specified plunger (normally with a diameter of 0.5 inch) to depress the surface of the gel by 4 mm without breaking it at a specified temperature. The number of grams is called the Bloom value, and most gelatins are between 30 and 300 g Bloom. The higher a Bloom value, the higher the melting and gelling points of a gel, and the shorter its gelling times. This method is most often used on soft gels. To perform the Bloom test on gelatin, a 6.67% gelatin solution is kept for 17-18 hours at 10 °C prior to being tested.

In one embodiment, the gelling step is carried out until the structured oil is in the range of low bloom, medium Bloom or High Bloom, wherein the respective Bloom numbers for each range and the average molecular mass of the equivalent gelatin solution s provided in the following table

| **Category** | **Bloom number** | **Average molecular mass** |
|---|---|---|
| Low Bloom | 50-125 | 20,000-25,000 |
| Medium Bloom | 175-225 | 40,000-50,000 |
| High Bloom | 225-325 | 50,000-100,000 |

The method according to claim 1 further comprising a step wherein the structured oil obtained in step (ii) is cooled at a temperature lower than the temperature at which the processing step (ii) is carried out.

In one embodiment, the aqueous solution containing the emulsifier, the hydrocolloid and/or hydrocolloids and which are used in step (i) of the method according to the invention are obtained by vigorous shaking at a speed of 3.000-10.000 rpm.

In another embodiment, the oil and the aqueous solution, in those embodiments when a single solution containing the hydrocolloid and the emulsifier or emulsifiers is used, are mixed at a w/w ratio of 0,1%-70%. In another embodiment, when several aqueous solutions are used each containing one of the different components (emulsified, first hydrogen and second hydrogel), the oil is mixed at a w/w ratio of 0,1%-70% with respect with the combined content of the different aqueous solutions.

In another embodiment, the aqueous solution or solutions are combined with the oil by shaking at a temperature of 15-100°C, more preferably 70-95°C, during sufficient until the emulsion is formed. Typically, step (ii) is carried out during 5-30 min, although shorter or longer times are possible depending on the nature of the oil, the aqueous solutions, the hydrocolloid and emulsifier used in the process. In another embodiment, the processing of the mixture in step (ii) is carried out at about 3.000-10.000 rpm.

In another embodiment, the method according to the invention is carried out under conditions in which the aqueous solution containing the first hydrocolloid is provided at a temperature of between 85-87°C, wherein the aqueous solution containing the second hydrocolloid is provided at a temperature of between 75-80°C or wherein the single aqueous solution containing the first and second hydrocolloids is provided at a temperature of about 80-85°C.

Once step (ii) has been completed and the structured oil has been formed, the emulsion will have to be processed for storage. Typically, the emulsion can be cooled. In one embodiment, the structured oil is cooled down at temperatures below 40°C, in particular in those cases in which gelatin is used as emulsifier or as one hydrocolloids. In another embodiment, the structured oil is cooled at a final temperature of 0-10 °C, which is the temperature at which the oil can be stored. In another embodiment, the structured oil is kept at room temperature, more preferably at about 10-30°C. In another embodiment, the structured oil is kept frozen, preferably at temperatures between -50 and -10 °C.

In yet another embodiment, the method according to the invention does not include a step of cross-linking the first or second hydrocolloid.

As used herein, the term "cross-linking" or "polymerization" refers to at least one reaction that consumes at least one functional group in one hydrogel molecule to create at least one chemical linkage between at least two distinct hydrogel molecules. In the majority of cases, a cross-link is a covalent structure or covalent bond, but the term may also describe sites of weaker chemical interactions, portions of crystallites, and even physical interactions and entanglements. The cross-linking can alter the physical and mechanical properties of the polymer. Cross-linking may be formed by chemical reactions that are initiated by heat, pressure, change in pH, and/or radiation, with or without the presence of a cross-linking agent and/or catalyst. A polymerization or cross-linking reaction may consume between about 0 percent and about 100 percent of the at least one functional group available in the system. In one embodiment, the hydrocolloids molecules are not substantially cross-linked. In another embodiment, the hydrocolloids are cross-linked at a degree of less than 10%, less than 9%, less than 8%, less than 7%, less than 6%, less than 5%, less than 4%, less than 3%, less than 2%, less than 1%, less than 0,9%, less than 0,8%, less than 0,7%, less than 0,6%, less than 0,5%, less than 0,4%, less than 0,3%, less than 0,2% or less than 0,1%.

In another embodiment, the structured oil is not dehydrated or only minimally dehydrated after the formation of the o/w emulsion in step (ii). The term "dehydration" refers to a partial and/or complete removal of the water of the structured oil. In preferred embodiment, the structured oil contains at least 10 percent, at least 20 percent, at least 30 percent, at least 40 percent, at least 50 percent, at least 60 percent, at least 70 percent, at least 80 percent, at least 90 percent, at least 99 percent or 100 percent of the water originally contained in the structured oil after step (ii). The dehydration of the biomass can be performed by any suitable method, for example, by freeze-drying or by spray-drying.

### Structured oil

In another aspect, the invention relates to a structured oil system in the form of an o/w emulsion comprising a lipid phase (o) and a water phase (w), the lipid phase being dispersed inside the water phase wherein the oil phase is stabilized in the water phase by an emulsifier and at least a first hydrocolloid dissolved or dispersed in the water phase.

In another embodiment, the invention relates to a structured oil which has been obtained or is obtainable by the method according to the present invention.

The terms "emulsion", "o/w emulsion", "emulsifier" and "hydrocolloid" have been defined in the context of the method for obtaining the structured oil and are equally applicable to the structured oil according to the invention.

In one embodiment, the oil phase in the structured oil system is a vegetable oil, an algal oil, a microbial oil or a fish oil. Suitable vegetable oil, algal oil, microbial oil or a fish oil for use in the structured oil system are as defined above in the context of the method according to the invention.

In one embodiment, the emulsifier is also an hydrocolloid.

In another embodiment, the at least one hydrocolloid is a denatured polypeptide. In yet another embodiment, the denatured polypeptide is gelatine or pea protein.

In another embodiment, the hydrocolloid of the first hydrocolloid in those cases wherein more than one hydrocolloid are used is a polysaccharide. In a still more preferred embodiment, the polysaccharide is xanthan gum. In yet another embodiment, if a second hydrocolloid is used, then the second hydrocolloid is a polysaccharide.

Suitable combinations of emulsifier, first hydrocolloid, second hydrocolloid and optional additional hydrocolloid are as shown above in Table 1. Preferred structured oil systems according to the invention are shown above in Table 2.

The oil phase in the structured oil system preferably contains unsaturated fatty acids, including both polyunsaturated fatty acids and monounsaturated fatty acids. These fatty acids are prone to oxidation, leading to the production of reactive oxygen species and metabolites that may affect the efficacy of PUFAs. The oxidation causes deleterious changes in flavor, taste, color, texture, and possibly safety of foods. In order to reduce the oxidation, the structured oil compositions according to the present invention are further comprise one or more antioxidant compound or a composition comprising antioxidant compounds.

Examples of suitable antioxidants for embodiments of this application include, but are not limited to, vitamins, vitamin cofactors, minerals, hormones, carotenoids, carotenoid terpenoids, non-carotenoid terpenoids, flavonoids, flavonoid polyphenolics (e.g., bioflavonoids), flavonols, flavones, phenols, polyphenols, esters of phenols, esters of polyphenols, nonflavonoid phenolics, isothiocyanates, and combinations thereof. In some embodiments, the antioxidant is vitamin A, vitamin C, vitamin E, ubiquinone, mineral selenium, manganese, melatonin, α-carotene, β-carotene, lycopene, lutein, zeanthin, crypoxanthin, reservatol, eugenol, quercetin, catechin, gossypol, hesperetin, curcumin, ferulic acid, thymol, hydroxytyrosol, tumeric, thyme, olive oil, lipoic acid, glutathinone, gutamine, oxalic acid, tocopherol-derived compounds, butylated hydroxyanisole (BHA), butylated hydroxytoluene (BHT), ethylenediaminetetraacetic acid (EDTA), tert-butylhydroquinone, acetic acid, pectin, tocotrienol, tocopherol, coenzyme Q10, zeaxanthin, astaxanthin, canthaxantin, saponins, limonoids, kaempfedrol, myricetin, isorhamnetin, proanthocyanidins, quercetin, rutin, luteolin, apigenin, tangeritin, hesperetin, naringenin, erodictyol, flavan-3-ols (e.g., anthocyanidins), gallocatechins, epicatechin and its gallate forms, epigallocatechin and its gallate forms (ECGC) theaflavin and its gallate forms, thearubigins, isoflavone, phytoestrogens, genistein, daidzein, glycitein, anythocyanins, cyaniding, delphinidin, malvidin, pelargonidin, peonidin, petunidin, ellagic acid, gallic acid, salicylic acid, rosmarinic acid, cinnamic acid and its derivatives (e.g., ferulic acid), chlorogenic acid, chicoric acid, gallotannins, ellagitannins, anthoxanthins, betacyanins and other plant pigments, silymarin, citric acid, lignan, antinutrients, bilirubin, uric acid, R-a-lipoic acid, N-acetylcysteine, emblicanin, apple extract, apple skin extract (applephenon), rooibos extract red, rooibos extract, green, hawthorn berry extract, red raspberry extract, green coffee antioxidant (GCA), aronia extract 20 percent, grape seed extract (VinOseed), cocoa extract, hops extract, mangosteen extract, mangosteen hull extract, cranberry extract, pomegranate extract, pomegranate hull extract, pomegranate seed extract, hawthorn berry extract, pomella pomegranate extract, cinnamon bark extract, grape skin extract, bilberry extract, pine bark extract, pycnogenol, elderberry extract, mulberry root extract, wolfberry (gogi) extract, blackberry extract, blueberry extract, blueberry leaf extract, raspberry extract, turmeric extract, citrus bioflavonoids, black currant, ginger, acai powder, green coffee bean extract, green tea extract, and phytic acid, or combinations thereof. In alternate embodiments, the antioxidant is a synthetic antioxidant such as butylated hydroxytolune or butylated hydroxyanisole, for example. Other sources of suitable antioxidants for embodiments of this application include, but are not limited to, fruits, vegetables, tea, cocoa, chocolate, spices, herbs, rice, organ meats from livestock, yeast, whole grains, or cereal grains.

Particular antioxidants belong to the class of phytonutrients called polyphenols (also known as "polyphenolics"), which are a group of chemical substances found in plants, characterized by the presence of more than one phenol group per molecule. Suitable polyphenols for embodiments of this application include catechins, proanthocyanidins, procyanidins, anthocyanins, quercerin, rutin, reservatrol, isoflavones, curcumin, punicalagin, ellagitannin, hesperidin, naringin, citrus flavonoids, chlorogenic acid, other similar materials, and combinations thereof.

In particular embodiments, the antioxidant is a catechin such as, for example, epigallocatechin gallate (EGCG). Suitable sources of catechins for embodiments of this application include, but are not limited to, green tea, white tea, black tea, oolong tea, chocolate, cocoa, red wine, grape seed, red grape skin, purple grape skin, red grape juice, purple grape juice, berries, pycnogenol, and red apple peel.

In some embodiments, the antioxidant is chosen from proanthocyanidins, procyanidins or combinations thereof. Suitable sources of proanthocyanidins and procyanidins for embodiments of this application include, but are not limited to, red grapes, purple grapes, cocoa, chocolate, grape seeds, red wine, cacao beans, cranberry, apple peel, plum, blueberry, black currants, choke berry, green tea, sorghum, cinnamon, barley, red kidney bean, pinto bean, hops, almonds, hazelnuts, pecans, pistachio, pycnogenol, and colorful berries.

In particular embodiments, the antioxidant is an anthocyanin. Suitable sources of anthocyanins for embodiments of this application include, but are not limited to, red berries, blueberries, bilberry, cranberry, raspberry, cherry, pomegranate, strawberry, elderberry, choke berry, red grape skin, purple grape skin, grape seed, red wine, black currant, red currant, cocoa, plum, apple peel, peach, red pear, red cabbage, red onion, red orange, and blackberries.

In some embodiments, the antioxidant is chosen from quercetin, rutin or combinations thereof. Suitable sources of quercetin and rutin for embodiments of this application include, but are not limited to, red apples, onions, kale, bog whortleberry, lingonberrys, chokeberry, cranberry, blackberry, blueberry, strawberry, raspberry, black currant, green tea, black tea, plum, apricot, parsley, leek, broccoli, chili pepper, berry wine, and ginkgo.

In some embodiments, the antioxidant is reservatrol. Suitable sources of reservatrol for embodiments of this application include, but are not limited to, red grapes, peanuts, cranberry, blueberry, bilberry, mulberry, Japanese Itadori tea, and red wine.

In particular embodiments, the antioxidant is an isoflavone. Suitable sources of isoflavones for embodiments of this application include, but are not limited to, soy beans, soy products, legumes, alfalfa sprouts, chickpeas, peanuts, and red clover.

In some embodiments, the antioxidant is curcumin. Suitable sources of curcumin for embodiments of this application include, but are not limited to, turmeric and mustard.

In particular embodiments, the antioxidant is chosen from punicalagin, ellagitannin or combinations thereof. Suitable sources of punicalagin and ellagitannin for embodiments of this application include, but are not limited to, pomegranate, raspberry, strawberry, walnut, and oak-aged red wine.

In some embodiments, the antioxidant is a citrus flavonoid, such as hesperidin or naringin. Suitable sources of citrus flavonoids, such as hesperidin or naringin, for embodiments of this application include, but are not limited to, oranges, grapefruits, and citrus juices.

In particular embodiments, the antioxidant is chlorogenic acid. Suitable sources of chlorogenic acid for embodiments of this application include, but are not limited to, green coffee, yerba mate, red wine, grape seed, red grape skin, purple grape skin, red grape juice, purple grape juice, apple juice, cranberry, pomegranate, blueberry, strawberry, sunflower, Echinacea, pycnogenol, and apple peel.

It will be understood that the antioxidant or antioxidants may be added to the structured oil once it has been prepared or can be included in one of the phases used in the preparation of the structured oil. The incorporation of the antioxidant in the aqueous phase or in the lipid phase will depend on the solubility of the antioxidant in each of the phases. For instance, in the case wherein the antioxidant is tocopherol (vitamin E), the antioxidant is included in the oil phase.

### Foodstuffs containing structured oil

The structured oil of the invention presents a solid or semisolid consistency which makes it suitable for the completely replacement of animal and vegetable fats in food products, while retaining the organoleptic properties of the original product such as desirable taste and mouth feel, and appropriate texture. For the purposes of the invention, the fact that the emulsion has "semisolid" consistency means that it has the consistency between a solid and a liquid at room temperature. More particularly, the emulsion of the invention has a viscosity from 50000 to 150000 mPa.s, more preferably from 90000 to 130000 mPas (as measured with a Brookfield, Helipath S94, 10 rpm, 2 degrees C).

Thus, in another embodiment, the invention relates to a foodstuff that comprises the structured oil according to the invention.

The term "foodstuff refers to any substance or product of any nature, solid or liquid, natural or processed which due to its characteristics, applications, components, preparation and state of preservation, can usually or ideally be used for some of the following purposes: a) as normal nutrition for human beings or animals or as pleasurable foods; or b) as dietetic products, in especial cases of human or animal food; thus, the definition broadly covers all the natural materials and finished products of any origin which, separately or conveniently mixed with one another, are suitable in the diet of human beings or animals. A ready-to-eat foodstuff is that which does not need to be diluted by means of an aqueous solution suitable for consumption for example. In principle, the ingredients present in a ready-to- eat foodstuff are balanced and there is no need to add additional ingredients to the foodstuff to make it ready to eat, such considered by a person skilled in the art. A concentrated foodstuff is that in which one or more ingredients are present at a higher concentration than in a ready-to-eat foodstuff, therefore for use it is necessary to dilute it by means of an aqueous solution suitable for consumption for example. Non-limiting, illustrative examples of foods provided by this invention include dairy products as milk, yogurts, margarines; drinks as juices and sport drinks; foods like biscuits, breads, cereals, pasta, sauces, etc.

In a particular embodiment, the foodstuff comprises between 0,001% and 99,998% by weight of the active structured fat of the invention. In preferred embodiments, the food product may contain at least 1 percent by weight, at least 10 percent by weight, at least 15 percent by weight, at least 20 percent by weight, at least 25 percent by weight or at least 30 percent, at least 40 percent, at least 50 percent, at least 60 percent, at least 70 percent, at least 80 percent, or at least 90 percent by weight of structured fat relative to the total weight of the product or a value in a range having any of those two values as endpoints.

In a preferred embodiment, the foodstuff according to the invention is selected from the group consisting of a spread, a shortening, a dairy product, a vegetable oil-based dairy alternative, a bakery product and a meat or a processed meat product.

In one embodiment, the foodstuff is a spread.

As used herein, the term "spread" means a food product that is literally spread, typically with a knife onto another food item such as e.g. bread or crackers. Preferred spreads of the present invention include chocolate spreads, nut-based spreads (peanut butter spread, almond butter spread, hazelnut spread), speculoos spreads, cheese or cream-cheese spreads, and savoury spread.

In one embodiment, the foodstuff is a dairy product such as milk beverage, fermented milk beverage, butter, cream, processed cheese, cheese processed food, etc. or a dairy product substitutes margarine, shortening, fat spread, non-dairy creamer, coffee cream, whipped cream, etc. In another embodiment, the food product is a cold confectionery such as ice cream, jelly, pudding, etc.) and the like. In one embodiment, the food product of the invention may be, or comprise cheese. The cheese may be, for instance, a hard cheese or a soft cheese. For instance, it may be a cheddar, stilton, camembert, gouda, edam, Roquefort, mozzarella, parmesan or other cheese. In one preferred instance, the cheese is a cheddar cheese. The food product may be a cheese spread and/or processed cheese. In one instance, the cheese is a smoked cheese and in particular a smoked processed cheese. The food product may be a formed cheese product, such a cheese triangle or slice. The cheese may be a cheese spread. The cheese may be a hard or soft cheese. In further instances, the food product is, or comprises, a fungal cheese. In a preferred instance, the cheese is a blue or white fungal cheese. The cheese may be a veined cheese. Suitable cheeses include Roquefort; Camembert; Danish Blue cheese; Creamy Blue; Stilton; Cabrales, Blue Brie, Cambozola; Fourme d'Ambert; Gorgonzola; Bleu d'Auvergne and Cibosano and other mould containing cheeses. The cheese may be, for instance, a cheese made from, or comprising, cow, sheep, goat or buffalo milk. The cheese may be pasteurised or unpasteurised. The cheese may have been subjected to UHT treatment. Any of the compositions discussed herein may have been pasteurised or UHT treated or may be unpasteurised.

In another embodiment, the foodstuff according to the invention is a vegetable oil-based dairy alternative such as almond milk, chestnut milk, pecan milk, hazelnut milk, cashew milk, pine nut milk, and walnut milk as well as yogurts prepared using any of the above milks and other types of fermented milk products.

The structured oil according to the present invention can be used as an additive in meat products as a fat replacement, a moisture retention aid, a texture enhancer, a mouthfeel enhancer, or any combination thereof. Accordingly, in one embodiment, the foodstuff is a meat product, which includes, without limitation, sausages, burgers, hot dogs, jerkies, pet food and pet treats, etc., which may contain any known type of meat, such as poultry (chicken, turkey, Cornish game hen, etc.), beef, pork, lamb, rabbit, venison, buffalo, etc., or any combination thereof and vegetarian alternatives to traditional meat products (e.g., "veggie" burgers, sausages, nuggets, etc.).

In various aspects, the meat or processed meat product according to the invention further comprises beef, chicken, turkey, pork, lamb, rabbit, venison, game, buffalo, horse, plant proteins, fermented proteins, shell-fish (e.g., scallops, crab, lobster, etc.), fish, imitations of the foregoing, or combinations thereof. The meat or meat analog composition may further comprise plant proteins, such as, for example, peas, chick peas, beans, lentils, legumes, mushrooms, soy, peanut, rapeseed meal, grains (e.g., quinoa), and the like and combinations thereof. The meat or processed meat product n may further comprise fermented proteins, such as, for example, tofu or Quorn. In additional embodiment, the meat or processed meat product is in the form of a sausage, a burger, a kebab, a gyro, a shwarma, a patty, a cake, a loaf, a nugget, a strip, a hot dog, a deli product, a jerky, a pet food, a pet treat, a processed meat, an emulsified meat, or combinations thereof, or imitations thereof, and wherein the meat or processed meat product is made with beef, chicken, turkey, pork, lamb, horse, buffalo, venison, veal, game, fowl, plant proteins. The processed meat product, as used herein, also refers to imitations of meats or meat analogs such as such as vegetarian or vegan products comprising tofu, tempeh, seitan, beans, legumes, grain, or a combination thereof, in a form mimicking burgers, hot dogs, nuggets, etc.

In one embodiment, the foodstuff is a bakery product, such as a dough, a batter, and baked food items including, for example, fine bakery wares, breakfast cereals, cakes, cheesecakes, pies, cupcakes, cookies, bars, breads, rolls, biscuits, muffins, pastries, scones, croutons, crackers, sweet goods, snack cakes, pies, granola/snack bars, and toaster pastries; candy; hard confectionery; chocolate and other confectionery; chewing gum.

In another embodiment, the food product is a liquid food product or beverage. Beverages may include any drink which comprises fat compounds and may include dairy and non-dairy drinks. The formulation of suitable beverages is well-known in the art and includes, without limitation, milks, energy drinks, infant formula, carbonated drinks, teas, liquid meals, fruit juices, fruit-based drinks, vegetable-based drinks; multivitamin syrups, meal replacers, medicinal foods, and syrups; powdered beverage mixes. The foodstuff of the invention may also be something for addition to a beverage, such as a cream or cream substitute, for instance in a single serving container.

In one embodiment, the foodstuff is a filling.

As used herein, the term "filling" means an edible substance or mixture used to fill a cavity in another food item. Examples of fillings are peanut butter filling, praline filling, bonbon filling, caramel filling, butter cream filling, cereal filling, fillings for extruded snacks, fillings for chocolate bars, cheese or cheese cream fillings, fillings for jellies or chewing gums. Preferably, the filling of the present invention is a confectionery filling.

In another embodiment, the foodstuff is a coating or a spread.

As used herein, the term "coating" means a layer of an edible substance or mixture which is applied as a layer onto a food item. The layer can be applied as a liquid or as a solid. In some embodiments, the coating is applied to only portions of the food item. In other embodiments, the coating can completely encase the food item and thus encapsulate the food item. One example of a coating is icing or frosting which is a sweet, often creamy, glaze typically made of sugar with a liquid (water or milk) that is often enriched with ingredients such as butter, egg whites, cream cheese or flavorings.

In additional embodiments, the foodstuff is a mayonnaise, a salad dressing, a marinade, an aioli, a sandwich spread, a vegetable spread, a vegetable shortening, a vinaigrette, a condiment, a topping, a cheese, a yogurt, an ice cream, a butter, a margarine, a cream, a milk, a gravy, a fruit butter, a nut butter, a coffee beverage, a chocolate beverage, an imitation flavored beverage, a syrup, a soup or a sauce.

In one embodiment, the foodstuff is an edible ink.

The term "edible ink" as used herein, refers to a substance that can be shaped in the form of a meat fiber matrix that mimics the texture and flavor of real meat by additive manufacturing, preferably using a 3D printer. In some embodiments, the edible ink contains a mixture of protein, fiber, water, fat, coloring, flavor and aromas ingredients, wherein the fat is at least in part the structured oil according to the invention. One type of edible ink uses extrusion or micro-extrusion procedures of a protein matrix with a 3-10% of fat, including the structured fat of the invention, water (72-65%) and plant protein sources (20-23%), seaweed fiber and other ingredients (2-5%).

The food product according to the invention may contain one or more food additives which may be part of the lipid or water phase or be found in both phases.

The term "food additive", refers to an essentially pure compound or a multi component composition intended for or suitable for being added to food or feed. In particular it is a substance that by its intended use is becoming a component of a food or feed product or affects any characteristics of a food or feed product. The term is intended to mean any of the compounds listed in and corresponding to the Codex Alimentarius of the FAO/WHO - version 2013. Exemplary food additives suitable for being included in the structured oil according to the invention include antifreezing agent and/or an cryoprotective agents, aroma or flavouring compounds, food dyes and sweeteners

Suitable antifreezing agent and/or a cryoprotective agents include, within limitation, glycerol as antifreezing agent and inverted sugar as cryoprotectant. Suitable agents having antifreezing and/or a cryoprotective activity include propylenglycol, sorbitol, sucrose, glucose, trehalose, hydroxyethyl starch, guar gum and the like.

The term "aroma", "flavouring compound" or "flavorant", as used herein, has the usual meaning in the art in that it is a compound that imparts a hedonic effect, i.e. is able to impart or modify in a pleasant way the taste of a preparation, and not just as imparting a taste. The term includes both flavoring compounds as such as well as flavor precursors. i.e. compounds that are converted into flavoring compounds when the food is further process, i.e. by heating.

Suitable flavor precursors include sugars, sugar alcohols, sugar derivatives, free fatty acids, triglycerides, alpha-hydroxy acids, dicarboxylic acids, amino acids and derivatives thereof, nucleosides, nucleotides, vitamins, peptides, phospholipids, lecithin, pyrazine, creatine, pyrophosphate and organic molecules. For example, sugars, sugar alcohols, sugar acids, and sugar derivatives can include glucose, fructose, ribose, sucrose, arabinose, glucose-6-phosphate, fructose-6-phosphate, fructose 1,6-diphosphate, inositol, maltose, mannose, glycerol, molasses, maltodextrin, glycogen, galactose, lactose, ribitol, gluconic acid, glucuronic acid, amylose, amylopectin, or xylose. Free fatty acids can include caprylic acid, capric acid, lauric acid, myristic acid, palmitic acid, palmitoleic acid, stearic acid, oleic acid, linoleic acid, alpha linolenic acid, gamma linolenic acid, arachidic acid, arachidonic acid, behenic acid, eicosapentaenoic acid, petroselinic acid or erucic acid. Triglycerides can include fatty acid esters of caprylic acid, capric acid, lauric acid, myristic acid, palmitic acid, palmitoleic acid, stearic acid, oleic acid, linoleic acid, alpha linolenic acid, gamma linolenic acid, arachidic acid, arachidonic acid, behenic acid, eicosapentaenoic acid, petroselinic acid or erucic acid. Amino acids and derivatives thereof can include cysteine, cystine, a cysteine sulfoxide, allicin, selenocysteine, methionine, isoleucine, leucine, lysine, phenylalanine, threonine, tryptophan, 5-hydroxytryptophan, valine, arginine, histidine, alanine, asparagine, aspartate, glutamate, glutamine, glycine, proline, serine, tyrosine, ornithine, carnosine, citrulline, carnitine, ornithine, theanine, and taurine. Phospholipids can include a plurality of amphipathic molecules comprising fatty acids, glycerol and polar groups. The fatty acids are selected from the group consisting of oleic acid, palmitoleic acid, palmitic acid, myristic acid, lauric acid, myristoleic acid, caproic acid, capric acid, caprylic acid, pelargonic acid, undecanoic acid, linoleic acid, 20:1 eicosanoic acid, arachidonic acid, eicosapentanoic acid, docosohexanoic acid, 18:2 conjugated linoleic acid, conjugated oleic acid, or esters of: oleic acid, palmitoleic acid, palmitic acid, myristic acid, lauric acid, myristoleic acid, caproic acid, capric acid, caprylic acid, pelargonic acid, undecanoic acid, linoleic acid, 20:1 eicosanoic acid, arachidonic acid, eicosapentanoic acid, docosohexanoic acid, 18:2 conjugated linoleic acid, or conjugated oleic acid, or glycerol esters of oleic acid, palmitoleic acid, palmitic acid, myristic acid, lauric acid, myristoleic acid, caproic acid, capric acid, caprylic acid, pelargonic acid, undecanoic acid, linoleic acid, 20:1 eicosanoic acid, arachidonic acid, eicosapentanoic acid, docosohexanoic acid, 18:2 conjugated linoleic acid, or conjugated oleic acid, or triglyceride derivatives of oleic acid, palmitoleic acid, palmitic acid, myristic acid, lauric acid, myristoleic acid, caproic acid, capric acid, caprylic acid, pelargonic acid, undecanoic acid, linoleic acid, 20:1 eicosanoic acid, arachidonic acid, eicosapentanoic acid, docosohexanoic acid, 18:2 conjugated linoleic acid, or conjugated oleic acid. In some embodiments, the polar groups are selected from the group consisting of choline, ethanolamine, serine, phosphate, glycerol-3-phosphate, inositol and inositol phosphates.

The term "food dye", as used herein, refers to compounds that imparts color when it is added to food or drink. The term includes natural coloring agents such as turmeric or beet juice, annatto (E160b), caramel coloring (E150a-d), carmine (E120), elderberry juice (E163), lycopene (E160d), paprika (E160c) and turmeric (E100) as well as artificial coloring agents such as an azo dye, triphenylmethane, xanthene, quinine, indigoid, titanium dioxide, red #3, red #40, blue #1, or yellow #5, or any combination of natural and/or artificial coloring agents.

The term "sweeteners", as used herein, include, without limitation sorbitol, mannitol, Acesulfame K, Sucralose, Saccharines, Aspartame, Cyclamates, Isomaltose, Taumatine, Neohesperidine DC, Neotame, Aspartame and Acesulfame salts, Maltitol, Lactitol, Xylitol, Erythritol.

The term "aroma" or "flavouring compounds", as used herein, includes agents conferring flavours such as vanilla, raspberry, orange, mint, citrus, strawberry, apricot, lavender flavours, etc, and any other fruit, nutty or flower flavouring agent, among others

In some embodiments, the foodstuff according to the invention are enriched in one or more vitamins. Vitamins are organic compounds that the human body needs in small quantities for normal functioning. Suitable vitamins and their alternative chemical names are provided in the accompanying parentheses which follow include, vitamin A (retinol, retinaldehyde), vitamin D (calciferol, cholecalciferol, lumisterol, ergocalciferol, dihydrotachysterol, 7-dehydrocholesterol), vitamin E (tocopherol, tocotrienol), vitamin K (phylloquinone, naphthoquinone), vitamin B1 (thiamin), vitamin B2 (riboflavin, vitamin G), vitamin B3 (niacin, nicotinic acid, vitamin PP), vitamin B5 (pantothenic acid), vitamin B6 (pyridoxine, pyridoxal, pyridoxamine), vitamin B7 (biotin, vitamin H), vitamin B9 (folic acid, folate, folacin, vitamin M, pteroyl-L-glutamic acid), vitamin B12 (cobalamin, cyanocobalamin), and vitamin C (ascorbic acid).

Various other compounds have been classified as vitamins by some authorities. These compounds may be termed pseudo-vitamins and include, but are not limited to, compounds such as ubiquinone (coenzyme Q10), pangamic acid, dimethylglycine, taestrile, amygdaline, flavanoids, para-aminobenzoic acid, adenine, adenylic acid, and s-methylmethionine. As used herein, the term vitamin includes pseudo-vitamins.

In some embodiments, the vitamin is a fat-soluble vitamin chosen from vitamin A, D, E, K and combinations thereof. In other embodiments, the vitamin is a water-soluble vitamin chosen from vitamin B I, vitamin B2, vitamin B3, vitamin B6, vitamin B12, folic acid, biotin, pantothenic acid, vitamin C and combinations thereof.

In some embodiments, the foodstuff according to the invention contains a preservative agent. The preservative is chosen from antimicrobials, antienzymatics or combinations thereof. Non-limiting examples of antimicrobials include sulfites, propionates, benzoates, sorbates, nitrates, nitrites, bacteriocins, salts, sugars, acetic acid, dimethyl dicarbonate (DMDC), ethanol, and ozone.

According to a particular embodiment, the preservative is a sulfite. Sulfites include, but are not limited to, sulfur dioxide, sodium bisulfite, and potassium hydrogen sulfite.

According to another particular embodiment, the preservative is a propionate. Propionates include, but are not limited to, propionic acid, calcium propionate, and sodium propionate.

According to yet another particular embodiment, the preservative is a benzoate. Benzoates include, but are not limited to, sodium benzoate and benzoic acid.

In another particular embodiment, the preservative is a sorbate. Sorbates include, but are not limited to, potassium sorbate, sodium sorbate, calcium sorbate, and sorbic acid.

In still another particular embodiment, the preservative is a nitrate and/or a nitrite. Nitrates and nitrites include, but are not limited to, sodium nitrate and sodium nitrite.

In yet another particular embodiment, the at least one preservative is a bacteriocin, such as, for example, nisin.

In another particular embodiment, the preservative is ethanol or ozone.

In another embodiment, the preservative is an antienzymatic. Non-limiting examples of antienzymatics suitable for use as preservatives in particular embodiments of the application include ascorbic acid, citric acid, and metal chelating agents such as ethylenediaminetetraacetic acid (EDTA).

### Uses of the structured fat according and process for the manufacturing of a foodstuff using the structured fat

The structured fat according to the invention can be used in the preparation of foodstuff. Accordingly, in another embodiment, the invention relates to the use of the structured oil obtained by the method of the invention or the structured oil according to the invention for the preparation of a foodstuff.

In another embodiment, the invention relates to a method for the preparation of a foodstuff which comprises mixing the structured oil obtained by a method according to the invention or the structured oil according the invention with one or more edible ingredients.

The term foodstuff has been defined in detail previously and is used herein with the same meaning. It will be understood that the food product can be further processed once it has incorporated the structured fat system according to the invention. Accordingly, the food product, in particular when it is a meat product, can be sliced, thinly sliced, or diced or processed so as to obtain bite-size or a bite-size meat product. In another embodiment, the food product is refrigerated or frozen. In yet another embodiment, the food product is cooked, wherein the cooking comprises grilling, steaming, oven cooking, deep frying with plenty of oil, frying in a frying pan, or cooking in a microwave oven.

In yet another embodiment, the mixing of the structured oil obtained by a method according to the invention or of the structured oil according the invention with one or more edible ingredients is carried out by injecting the structured oil into the food product. In a more preferred embodiment, the food product is a meat product, a fish product, a processed meat product or a processed fish product. The injection results in the tenderization of the product. The injection is carried out inserting one or more needles into the product and injecting the structured fat according to the invention. The structured oil can be pumped into an injector apparatus which may include a plurality of needles configured to inject the liquid marinate under pressure directly into the tenderized product. As the product passes through the injector apparatus, the needles containing the liquid marinate can be repeatedly moved into and out of the product, injecting the marinate into the product with each penetration. The injection pressure of the marinate entering the product ejects the needles back out of the product, such that marinate may be injected as the needles are being ejected, thereby distributing the marinate throughout the thickness of the product. In some examples, the needles can be pushed about 90 percent of the way through each product cut. In embodiments, the penetration distance relative to the thickness of the product may vary, ranging from about 20 percent, 30 percent, 40 percent, 50 percent, 60 percent, 70 percent, 80 percent, 90 percent, 95 percent, or any range therebetween.

In another embodiment, the structured fat according to the invention is used as a fat replacement to decrease the content of trans- or saturated fat content in a food product. Accordingly, in another embodiment, the invention relates to the use of the structured oil obtained by a method of the invention or to the use of the structured oil of the invention for reducing the fat content of a foodstuff. It will be understood that the reduction of the trans- or saturated fat content in a food product can be an absolute reduction (the product contains less fat than the starting product) or a relative reduction (the product contains less trans- or saturated fat than the starting product). In the case of an absolute reduction, the structured oil according to the invention is used during the manufacturing process of the food product so that the structured oil is used instead of the trans- or saturated fat, so that the total content of trans- or saturated fat in the final product is lower than in the product which has been prepared using the trans- or saturated fat.. Accordingly, in one embodiment, the food product contains at least 10%, at least 20%, at least 30%, at least 40%, at least 50%, at least 60%, at least 70%, at least 80%, at least 90%, at least 95%, at least 96%, at least 97%, at least 98%, at least 99% less fat than the product containing trans- or saturated fat.

In other embodiments, the structured fat according to the invention is applied to the foodstuff so that the relative content of the trans- or saturated fat with respect to the total fat is decreased. Thus, in another embodiment, the structured fat is added to the foodstuff so that the ratio of trans- or saturated fat with respect to the total fat is decreased by at least 10%, at least 20%, at least 30%, at least 40%, at least 50%, at least 60%, at least 70%, at least 80%, at least 90%, at least 95%, at least 96%, at least 97%, at least 98%, at least 99% or more.

### Cosmetic compositions Process for the manufacturing of a cosmetic composition

Additionally, the invention relates to the use of the structured fat composition in the cosmetics area, more specifically in preparing cosmetic products with "pasty or creamy consistency". At present, there is a demand for the use of other fats, and even wax, in conjunction with vegetable oils, so that the lipidic composition can reach a fat consistency, that is, so that it can be solid at room temperature, since for the area of "Oils and Fats" one understands by "oils" products that are liquid at room temperature; one understands by "fats" products that are solid at room temperature, and one understands by "pasty (creamy)" products with a pasty characteristic similar to a milk cream.

Thus, the structured fat composition provided by the present invention represents an alternative to meet this demand, and it may be, for example, an intermediate product in the cases in which it is necessary to have a base from the liquid oils, but it is not possible due to the nature itself of these oils and, in these conditions, it is necessary to use an ingredient or product capable of structuring them, or emulsions in general, especially where it is necessary to use liquid vegetable oils that do not impart structure to the products formed.

Especially in the use in the cosmetic branch, the present invention enables the utilization of various oleaginous sources in products in which the lipid source should be in the pasty or even liquid form.

For the purpose of the present invention, one understands by "pasty or creamy consistency" the one similar to a milk cream, in which the texture values (Yield value) are lower than 50 g/cm2.

Besides the aspect of enabling the use of higher contents of liquid oils, the presence of the emulsifiers enables one to increase the consistency of these oils and to enhance the stability of the crystalline network of the cosmetic products, for instance, even with variation in temperature. Various cosmetic products are carried, especially by women, in bags, where the temperature varies much and may cause destabilization of the product, with appearance of the free oil. In this case, the emulsifier can act in a parallel manner and assume two functions: that of a structuring agent and hat of an emulsifier, with greater dispersion of the ingredient of the cosmetic product.

The invention is defined by the following aspects.
1. A method for obtaining a structured oil in the form of an oil-in-water (o/w) emulsion comprising the steps of
   (i) Contacting an oil with a emulsifier and at least one hydrocolloid, said emulsifier and said at least one hydrocolloid being provided in aqueous solution and
   (ii) Processing the mixture obtained in step (i) under conditions adequate for the dispersion of the oil in the aqueous solution to obtain the o/w emulsion.
2. The method according to aspect 1 further comprising a step wherein the structured oil obtained in step (ii) is processed under conditions adequate for the gelation of the structured oil.
3. The method according to aspect 1 wherein said gelation is carried out by changing the temperature of the o/w emulsion to a temperature which is lower than the gelling temperature of the aqueous solution containing the at least one hydrocolloid, by inducing cationic gelation between a negatively charge macromolecule and a positively charged counterion which or by lowering the pH to a value which is lower than the gelling pH of the aqueous solution containing the at least one hydrocolloid.
4. The method according to any of aspects 1 to 3 wherein a single hydrocolloid is used in step (i).
5. The method according to aspect 4 wherein the emulsifier and the single hydrocolloid are provided in a first and second aqueous solutions which are consecutively added to the oil.
6. The method according to aspect 5 wherein the emulsifier and the single hydrocolloid are provided in a single aqueous solution which is added to the oil.
7. The method according to aspect 1 wherein a first and second hydrocolloids are used in step (i).
8. The method according to aspect 7 wherein the emulsifier and the first and second hydrocolloids are provided in a first, second and third aqueous solutions which are consecutively added to the oil.
9. The method according to aspect 7 wherein the emulsifier is provided in a first aqueous solution and the first and second hydrocolloids are provided in a second aqueous solution which are added to the oil emulsifier or wherein the first and the second hydrocolloid are provided in a single aqueous solution which is added to the oil or wherein.
10. The method according to aspect 9 wherein:
   (i) The water content in the mixture obtained in step (i) is between 30 and 99% (p/p),
   (ii) The oil content in the mixture obtained in step (i) is between 0,1 and 70% (p/p),
   (iii) The emulsifier content in the mixture obtained in step (i) is between 0,1 and 10% (p/p) and/or
   (iv) The total hydrocolloid content in the mixture obtained in step (i) is between 0,1 and 20% (p/p), wherein preferably if two hydrocolloids are used, then the content of each hydrocolloid is of between 0,1 and 10% (p/p).
11. The method according to aspect 10 wherein if two hydrocolloids are used, then the content of each hydrocolloid is of between 0,1 and 10% (p/p) and/or the ratio between the first and second hydrocolloid is of 0,1:4 to 4:0,1.
12. The method according to any of aspects 1 to 11 wherein the oil, the emulsifier, the first and the second hydrocolloids are from a non-animal source, preferably a plant source, an algal source or a microbial source.
13. The method according to any of aspects 1 to 12 wherein the oil comprises a vegetable oil or an algal oil, or a microbial oil.
14. The method according to any of aspects 1 to 13 wherein the emulsifier is also an hydrocolloid.
15. The method according to aspect 14 wherein the emulsifier is a denatured polypeptide.
16. The method according to aspect 15 wherein the denatured polypeptide is gelatine or pea protein.
17. The method according to any of aspects 1 to 16 wherein the first hydrocolloid is a polysaccharide.
18. The method according to aspect 17 wherein the polysaccharide is selected from the group consisting of xanthan gum.
19. The method according to any of aspects 1 to 18 wherein if a second hydrocolloid is used, then the second hydrocolloid is a polysaccharide.
20. The method according to any of aspects 1 to 19 wherein the aqueous solution further comprises an antifreezing agent and/or an cryoprotective agent
21. The method according to any of aspects 1 to 20 wherein the oil is provided at a temperature of about 80-83°C.
22. The method according to any of aspects 1 to 21 wherein the aqueous solution containing the first hydrocolloid is provided at a temperature of between 85-87°C, wherein the aqueous solution containing the second hydrocolloid is provided at a temperature of between 75-80°C or wherein the single aqueous solution containing the first and second hydrocolloids is provided at a temperature of about 80-85°C.
23. The method according to any of aspects 1 to 22 wherein the processing step (ii) is carried out by shearing at a 1000 to 10.000 rpm.
24. The method according to any of aspects 1 to 23 wherein step (ii) is carried out at a temperature of between 70-80°C.
25. The method according to any of aspects 1 to 24 wherein the method does not include a step of cross-linking the first or second hydrocolloid.
26. The method according to any of aspects 1 to 24 wherein the structured oil is not dehydrated after the formation of the o/w emulsion.
27. A structured oil system in the form of an o/w emulsion comprising a lipid phase (o) and a water phase (w), the lipid phase being dispersed inside the water phase wherein the oil phase is stabilized in the water phase by an emulsifier and at least a first hydrocolloid dissolved or dispersed in the water phase.
28. The structured oil system according to aspect 27 wherein the oil in the oil phase is a vegetable oil, an algal oil or a microbial oil.
29. The structured oil system according to any of aspects 27 or 28 wherein the emulsifier is also an hydrocolloid.
30. The structured oil system according to any of aspects 27 to 29 wherein the at least one hydrocolloid is a denatured polypeptide.
31. The structured oil system according to aspect 30 wherein the denatured polypeptide is gelatine or pea protein.
32. The structured oil system according to any of aspects 27 to 31 wherein the first hydrocolloid is a polysaccharide.
33. The structured oil system according to aspect 32 wherein the polysaccharide is xanthan gum.
34. The structured oil system according to any of aspects 27 to 33 wherein if a second hydrocolloid is used, then the second hydrocolloid is a polysaccharide.
35. The structured oil system according to any of aspects 27 to 34 wherein the aqueous solution further comprises an antifreezing agent and/or an cryoprotective agent
36. The structured oil system according to any of aspects 27 to 35 wherein the lipid or the water phase contain one or more food additives.
37. The structured oil system according to aspect 36 wherein the food additive is selected from the group consisting of aroma or flavouring compounds, food dyes and sweeteners.
38. A foodstuff comprising a structured oil obtained by a method according to any of aspects 1 to 25 or the structured oil according to any of aspects 27 to 37, said foodstuff comprising one or more edible ingredients.
39. The foodstuff according to aspect 38 wherein the foodstuff is selected from the group consisting of a spread, a shortening, a dairy product, a vegetable oil-based dairy alternative, a bakery product, a processed meat product and an edible ink.
40. The foodstuff according to aspects 38 or 39 wherein the structured oil is used as coating or as filling of the foodstuff or is used as a spread.
41. Use of the structured oil obtained by a method according to any of aspects 1 to 26 or the structured oil according to any of aspects 26 to 39 for the preparation of a foodstuff.
42. Use of the structured oil obtained by a method according to any of aspects 1 to 25 or the structured oil according to any of aspects 26 to 36 for reducing the content of trans-unsaturated fats or of hydrogenated fats in a foodstuff, wherein the structured oil comprises a high content in unsaturated oils.
43. A process for the manufacture of a foodstuff characterized in that it comprises a step of mixing a structured oil obtained by a method according to any of aspects 1 to 27 or the structured oil according to any of aspects 28 to 37 with one or more edible ingredients.
44. The process according to aspect 43 wherein said mixing is carried out by injecting the structured oil into the foodstuff and wherein the foodstuff is a meat product or a process meat product.
45. A cosmetic composition comprising a structured oil obtained by a method according to any of aspects 1 to 27 or the structured oil according to any of aspects 28 to 37 and one more cosmetically-acceptable carrier.

The invention is described herein by way of the following examples which are merely illustrative and not limitative of the scope of the invention.

### EXAM PLES

### Example 1: Preparation of a structured oil according to the invention

This product consists an oil emulsion (55-58% w/w fat content) in water. The emulsifier is jelly, 0,2-3%, and the stabilizer is xanthan gum. The product was obtained by first adding gelatin hot solution (70-95°C) on hot oil (70-90°C) using a high speed mixing system followed by the addition of a xanthan gum hot solution (70-90°C). Mixing was continued until ail complete dispersion of xanthan gum solution was achieved and the emulsion was completed.

### MATERIALS

Material used for the preparation of the exemplary compositions are shown in Table 3

**Table 3: Reagents and suppliers used for the preparation of the exemplary compositions**

| **REAGENTS** | SUPPLIER |
|---|---|
| ***Sunflower oil** (High content in Oleic Acid* ≥*80%)* | ***COREYSA** Brand: Capicua Osuna (Sevilla, Spain)* |
| ***Water** (very weak mineralization, dry matter* = *27mg*/*L)* | ***Calidad PASCUAL, S.A.L.** Brand: BEZOYA Aranda de Duero (Burgos, Spain)* |
| ***Neutral Jelly*** | ***Mondelez International** Brand: Royal professional, Gelatina neutra. Madrid (Spain)* |
| ***Xanthan gum*** | ***DANISCO*** - ***DUPONT** Brand: Grinsted Xanthan MAS-HD Melle (France)* |
| ***Extract enriched in tocopherols** Tocopherols 50% (w*/*w), in Sunflower oil* | ***BTSA S.A. (Biotecnologias Aplicadas s.a)** Brand: Nutrabiol SF T50 Madrid (Spain)* |

### METHODS

The stock solutions used for the preparation of an exemplary composition are listed in Table 4.

**Table 4: Stock solutions used in the preparation of the exemplary compositions**

| **Ingredient** | **% (w/w)** |
|---|---|
| Water | 40-60% |
| *Capicua Sunflower oil (80% Oleic acid)* | 30-60% |
| *Royal Professional* - *Neutral Jelly* | 0,2-3% |
| *Grinsted Xanthan MAS-HD (Xanthan qum)* | 0,01-3% |
| *Nutrabiol SF T50* | 0,1-3% |

### 1.- Conditioning equipment and ingredients

### Xanthan gum reservoir (hot solution).

A xanthan gum stock solution is prepared because the solution is very thick and there are loses while handling it. By using a stock solution, the amounts needed can be weighted, resulting in more accurate measurement.

Heat water to 70-90°C in a beaker with stirring bar on heating-magnetic stirrer. Seal the beaker using aluminum foil to avoid water evaporation. At 70-90°C add xanthan gum very slowly and mix using a blender (maximum stirring speed), avoid aerating maintaining the blender head into the solution while you are mixing. Ensure all xanthan gum is dispersed, there must not be any lump, and the dispersion shows homogeny and is very thick. Preserve this solution into hot water bath (70-95°C) with a thermometer (for controlling the temperature), well-sealed with aluminum foil to avoid evaporation, and keep this solution not less 30min in the hot water bath (70-95°C) to ensure complete hydration of xanthan gum. After this time, weight xanthan gum from reservoir, in a hot beaker (250ml), and well-sealed with aluminum foil keep this solution in oven (70-95°C).

### Heating sunflower oil at 70-90°C.

Heat sunflower oil to 70-90°C in a beaker (2000ml) with stirring bar on heating-magnetic stirrer..

### Hydrating and heating neutral gelatin.

Weight in a beaker (250ml), add slowly neutral jelly with gently agitation until complete dispersion, seal with aluminum foil . After this, put the beaker with jelly dispersion on heating-magnetic stirrer adjusting plate temperature at 150°C (300rpm) and taking control of temperature using a thermometer. Seal the beaker using aluminum foil to avoid water evaporation. Heat this solution to 70-95°C.

### 2.- Mixing ingredients, packaging and storage.

### Mixing ingredients.

The following components are needed:
- Sunflower oil at 70-90°C.
- Neutral jelly solution at 70-95°C
- Xanthan gum solution at 70-95°C

We are going to prepare this product mixing in the sunflower oil beaker (2000ml). Turn off heating.Add *Nutrabiol SF T50* into hot oil. Mix very gently. At this point, pour slowly the gelatin solution (70-95°C) in hot oil (70-90°C), and mixing jelly solution and hot oil using the blender with maximum stirring (avoid aerating). Add the gelatin solution on hot oil, addition must be close to blender head for a well mixing. Ensure well mixing moving the blender into the mixture, avoid aerating maintaining the blender head into the solution while you are mixing, then solution become opaque white with light ivory color. Immediately, and maintaining the agitation, add slowly mixing the xanthan gum hot dispersion (70-90°C) . Ensure well mixing into the mixture, the dispersion becomes white, opaque and very thick (final temperature: 60-90°C).

When then mixing is finished, we pour the resulting product in clean food container, and keep it in a fridge (2-4 °C) not less 12-48 hour before to use.

Finished product appearance: White and opaque jelly.

The process for preparing the exemplary composition is schematically depicted in Figure 1.

### Example 2: Preparation of a structured oil according to the invention

This product consists an oil emulsion (40-60% w/w fat content) in water. The emulsifier are carrageneenans kappa and iota, 0,1-0,8% each one, and stabilizer is locust bean gum. The product is obtained by adding the oil (70-95°C) on water dispersion of carrageneenans, pea protein and locust bean gum at 70-90°C, using a high-speed mixing system. Mixing is continued until complete dispersion oil and the emulsion is completed.

### MATERIALS

Material used for the preparation of the exemplary compositions are shown in Table 5

**Table 5: Reagents and suppliers used for the preparation of the exemplary compositions**

| **REAGENTS** | SUPPLIER |
|---|---|
| ***Sunflower oil** (High content in Oleic Acid* ≥*80%)* | ***COREYSA*** Brand: Capicua Osuna (Sevilla, Spain) |
| ***Decalcified tap water*** | |
| ***Carrageenans kappa and Iota*** | ***Sample from EPSA, S.A.*** Torrent (Spain) |
| ***Locust bean gum*** | ***Sample from EPSA, S.A.*** Torrent (Spain) |
| ***Pea protein*** | ***Chemital (Masso group)*** Brand: Artisante Terrassa (Spain) |
| ***Extract enriched in tocopherols** Tocopherols 50% (w*/*w), in Sunflower oil* | ***BTSA S.A. (Biotecnologias Aplicadas s.a)*** Brand: Nutrabiol SF T50 Madrid (Spain) |

### METHODS

The stock solutions used for the preparation of an exemplary composition are listed in Table 7.

**Table 6: Stock solutions used in the preparation of the exemplary compositions**

| **Ingredient** | **% (w/w)** |
|---|---|
| Decalcified tap water | 40-60% |
| Sunflower oil (80% Oleic acid) | 40-60% |
| Carrageenan kappa | 0,2-0,8% |
| Carrageenan Iota | 0,2-0,8% |
| Pea protein | 0,1-2% |
| Locust beam gum | 0,1-0,5% |
| Potassium chloride | 0,1-0,5% |
| Calcium lactate pentahydrate | 0,1-0,5% |
| *Nutrabiol SF T50* | 0,1-3% |

### 1.- Conditioning equipment and ingredients

### Pea protein, Carrageenans and Locust bean gum reservoir (hot solution).

Pea protein, Carrageenans and Locust bean gum stock solution is prepared because the solution is very thick and there are loses while handling it. By using a stock solution, the amounts needed can be weighted, resulting in more accurate measurement.

Heat water to 70-90°C in a beaker with stirring bar on heating-magnetic stirrer adjusting plate temperature at 150°C (200rpm) and taking control of temperature using a thermometer. Seal the beaker using aluminum foil to avoid water evaporation. At 70-90°C add pea protein, carrageenans and gum successively and very slowly, mix using a blender (maximum stirring speed,), avoid aerating maintaining the blender head into the solution while you are mixing. Ensure pea protein, carrageneenans and gum are well dispersed, if so, you there must not be any lump, dispersion must show homogeny and be very thick. Preserve into hot water bath (80-95°C) with a thermometer (for controlling the temperature), well-sealed with aluminum foil to avoid evaporation. Keep in hot water bath (80-95°C) until to ensure complete hydration of protein, carrageneenans and gum. After this, weight from this reservoir the necessary dispersion in a beaker (2000ml) and keep it well-sealed in oven or water bath(80-95°C) until be used.

### Heating sunflower oil at 70-90°C.

Heat sunflower oil to 70-90°C in a beaker (500ml) with stirring bar on heating-magnetic stirrer and taking control of temperature.

### Potassium chloride and calcium lactate solutions.

Weight in a beaker (50ml) these salts separately, add 20ml of water to each one with gently agitation until complete solution, seal with aluminum foil. After this, put the beakers on heating-magnetic stirrer taking control of temperature using a thermometer. Heat these solutions to 70-95°C.

### 2.- Mixing ingredients, packaging and storage.

### Mixing ingredients.

The following components are needed:
- Sunflower oil at 70-90°C.
- Pea protein, carrageenans, and locust bean gum solution at 70-90°C
- *Potassium chloride and calcium lactate pentahydrate solutions* at 70-90°C

Add *Nutrabiol SF T50,* into hot oil and wash carefully the beaker with hot oil using a Pasteur pipette. Mix very gently. At this point, pour slowly hot oil (70-90°C) into the hot water solution of protein, carrageneenans and gum, mixing this solution and hot oil using the blender with maximum stirring (avoid aerating). When you pour hot oil on the water solution must be close to blender head for a well mixing. Ensure a well mixing moving the blender into the mixture, avoid aerating maintaining the blender head into the solution while you are mixing. Then, the mixture become milky, with very light ivory color. Immediately, and maintaining the agitation, add slowly hot solutions of salts ensuring a well mixing. The mixture must be white, opaque and very thick (final temperature: 60-90°C) and there must not be any oil on surface, then the emulsion is completed. When mixture is finished, pour the resulting product in clean food container, close it tightly and keep it in a fridge (2-6 °C) not less 12-48 hour before to use.
Product finished appearance: White opaque jelly.
Yield: 90-97%.

### Example 3: Exemplary compositions according to the invention

Compositions according to the present invention are summarized in Table 7

**Table 7: Exemplary compositions of the invention**

| **FORMULA** | **COMPOSITION** | **APPLICATIONS** | **CHARACTERISTICS** |
|---|---|---|---|
| **1** | High Oleic sunflower oil (30-60%) | Non-heat treated processed meat. | Solid gel Melts in boiling water and grill Bovine flavor |
| | Decalcified water (70,00-40,00%) | | |
| | Gelatine (0,5-4%) | Examples: Hamburgers, sausages, cured sausages | |
| | Xanthan gum (0,2-1%) | | |
| | Flavour (0,1-3%) | | |
| | Tocopherol enriched extract (0,1-3%) | | |
| **2** | High Oleic sunflower oil (45-50%) | Non-heat treated processed meat. | Solid gel, high gel strength Good resistance to boiling water and grill, does not melt |
| | Decalcified water (50,00-55,00%) | | |
| | Sodium alginate (0,1-0,5%) | Examples: Hamburgers Sausages Cured sausages | |
| | Pea protein 80% (0,2-1,50%) | | |
| | Calcium sulphate dihydrate (0,1-0,50%) | | |
| **3** | High Oleic sunflower oil (40,00-50,00%) | Heat and non-heat treated processed meat | Solid gel, high gel strength Good resistance to boiling water and grill, does not melt Light pink colour |
| | Decalcified water (50,00-60,00%) | | |
| | Sodium alginate (0,80-0,30%) | | |
| | Pea Protein 80% (0,5-3,00%) | Examples: Hamburgers, sausages, cured sausages, mortadella, etc | |
| | Guar gum (0,15-0,2%) | | |
| | Xanthan gum (0,03-0,01%) | | |
| | Calcium sulfate dihydrate (0,1-0,50%) | | |
| | Trisodium citrate dihydrate (0,01-0,40%) | | |
| | Carminic acid 12%, E-120, (0,20%) | | |
| **4** | High Oleic sunflower oil (50,00-60,00%) | Non-heat treated processed meat. | Solid gel Melts in boiling water and grill |
| | Decalcified water (40,00-60,00%) | | |
| | Agar-agar (0,5-1,00%) | Examples: Hamburgers, | |
| | Pea protein 80% (Chemital) (0,5-2,00%) | | |
| | Xanthan gum (0,10-0,5%) | | |
| | Tocopherol enriched extract (0,1-1,0%) E-120 (12%), (0,1-0,3%) | sausages, cured sausages Vegetal-based ink for plant-based product | |
| **5** | High oleic sunflower oil (40,00-60,00%) | Non-heat treated processed meat. | Solid gel Melts in boiling water and grill |
| | Decalcified waterv (60,00-40,00-%) | | |
| | kappa carrageenan (0,20-0,8%) | Examples: Hamburgers, sausages, cured sausages | |
| | Iota carrageenan (0,20-0,8%) | | |
| | Pea protein 80% (0,10-2,00%) | | |
| | Locust beam gum (0,1-0,5%) | | |
| | Potassium chloride (0,1-0,50%) | | |
| | Calcium lactate pentahydrate (0,1-0,5%) | | |
| | Tocopherol enriched extract (0,1-3,00%) | | |
| **6** | Decalcified water (50-95%) | Brine for injection of meat and fish | Liquid emulsion-gel for injection and slow gelification process inside the product. Good resistance to boiling water and grill, does not melt. |
| | High oleic sunflower oil (45,00-1,00%) | | |
| | Sodium Alginate (0,10-2,00%) | | |
| | Xanthan gum (0,02-0,50%) | | |
| | Pea protein (0,10-2,00%) | | |
| | Calcium citrate tetrahydrate (0,10-2,00%) | | |
| | Trisodium citrate dihydrate (0,10-2,00%) | | |
| | Citric acid monohydrate (0,02-1,00%) | | |
| | Tocopherol enriched extract (0,1-3,00%) | | |

## Claims

1. A method for obtaining a structured oil in the form of an oil-in-water (o/w) emulsion comprising the steps of
(i) Contacting an oil with a emulgent and at least one hydrocolloid, said emulsifier and said at least one hydrocolloid being provided in aqueous solution and
(ii) Processing the mixture obtained in step (i) under conditions adequate for the dispersion of the oil in the aqueous solution to obtain the o/w emulsion.

2. The method according to claim 1 wherein a first and a second hydrocolloids are used in step (i)

3. The method according to any of claims 1 or 2 wherein the emulsifier is also an hydrocolloid.

4. The method according to any of claims 1 to 3 wherein the emulsifier is a denatured polypeptide.

5. The method according to claim 4 wherein the denatured polypeptide is gelatine or pea protein.

6. The method according to any of claims 1 to 5 wherein the at least one hydrocolloid or the first hydrocolloid is a polysaccharide.

7. The method according to claim 6 wherein the polysaccharide is xanthan gum.

8. The method according to any of claims 2 to 7 wherein if a second hydrocolloid is used, then the second hydrocolloid is a polysaccharide.

9. The method according to any of claims 1 to 8 wherein the method does not include a step of cross-linking the first or second hydrocolloid.

10. The method according to any of claims 1 to 9 wherein the structured oil is not dehydrated after the formation of the o/w emulsion.

11. A structured oil system in the form of an o/w emulsion comprising a lipid phase (o) and a water phase (w), the lipid phase being dispersed inside the water phase wherein the oil phase is stabilized in the water phase by an emulsifier and at least a first hydrocolloid dissolved or dispersed in the water phase.

12. A foodstuff comprising a structured oil obtained by a method according to any of claims 1 to 10 or the structured oil according to claim 11, said foodstuff comprising one or more edible ingredients.

13. Use of the structured oil obtained by a method according to any of claims 1 to 10 or the structured oil according to claim 11 for reducing the content of trans-unsaturated fats or of hydrogenated fats in a foodstuff, wherein the structured oil comprises a high content in unsaturated oils.

14. A process for the manufacture of a foodstuff **characterized in that** it comprises a step of mixing a structured oil obtained by a method according to any of claims 1 to 10 or the structured oil according to claim 11 with one or more edible ingredients.

15. A cosmetic composition comprising a structured oil obtained by a method according to any of claims 1 to 10 or the structured oil according to claim 11 and one more cosmetically-acceptable carrier.
